# EUROPÄISCHE PATENTSCHRIFT

(11) **EP 3 506 733 B1**
(45) Veröffentlichungstag und Bekanntmachung des Hinweises auf die Patenterteilung: **10.11.2021**
(21) Anmeldenummer: 17781381.3
(22) Anmeldetag: 05.09.2017
(51) Int. Cl.: G01N 1/04, A01G 7/00, A01G 9/029, A01G 31/02

(54) **ANZUCHT- UND PROBENNAHME-VERFAHREN**
CULTIVATION AND SAMPLING METHOD FOR PLANTS
MÉTHODE DE CULTURE ET D'ÉCHANTILLONNAGE DE PLANTES

(30) Priorität: 05.09.2016 EP 16187274
(43) Veröffentlichungstag der Anmeldung: 10.07.2019
(73) Patentinhaber: Deutsche Saatveredelung AG, 59557 Lippstadt (DE)
(72) Erfinder: MICIC, Zeljko, 33154 Salzkotten (DE); RADTKE, Simon, 59494 Soest (DE)
(74) Vertreter: Wickord, Wiro
(86) Internationale Anmeldenummer: PCT/DE2017/100740
(87) Internationale Veröffentlichungsnummer: WO 2018/041309

(56) Entgegenhaltungen:
- WO-A1-2009/020766
- WO-A1-2012/096568
- WO-A1-2013/104801
- JP-A- 2008 118 963
- US-A1- 2013 180 171

## Beschreibung

Die Erfindung betrifft ein Anzucht- und Probennahme-Verfahren für Pflanzen. Die Pflanzen werden hierbei in Anzuchtbehältern herangezogen. Dann werden von den Pflanzen Proben entnommen zur phänotypischen Beschreibung und/oder molekularbiologischen Untersuchung. Nach der Analyse der Proben werden die entsprechend einer vorgegebenen Spezifikation besonders geeigneten Pflanzen selektiert und für die Weiterzucht verwendet.

Derartige Anzucht- und Probennahme-Verfahren sind in der Praxis seit vielen Jahren bekannt und verwendet. Üblicherweise erfolgt die Probennahme manuell mit Hilfe von Zangen und/oder Stanzwerkzeugen, mit denen Gewebe aus den herangezüchteten Pflanzen ausgestanzt wird. Die ausgestanzten Pflanzenteile, beispielsweise Blattgrün, werden dann in Probenbehälter eingebracht und der weiteren Untersuchung zugeführt. Das Vorgehen ist hierbei mithin sehr arbeitsintensiv und zeitaufwendig, da große Mengen von Pflanzen in einem mehrstufigen Entwicklungsprozess herangezüchtet, analysiert und selektiert werden müssen, bis schließlich eine Pflanze mit den gewünschten Eigenschaften bereitgestellt werden kann. Insbesondere aufgrund eines sehr hohen manuellen Arbeitsanteils ist das Verfahren prinzipiell fehleranfällig. Beispielsweise kann es vorkommen, dass Proben vertauscht beziehungsweise eine Zuordnung einer Probe zu einer Pflanze fehlerhaft erfolgt. Darüber hinaus besteht eine nicht unerhebliches Risiko, dass Proben verschiedener Pflanzen in unerwünschter Weise durchmischt werden und eine fehlerhafte Zuordnung der Analyseergebnisse zu einer Pflanze möglich ist.

Die JP 2008 118963 A offenbart ein Anzucht- und Probennahme-Verfahren für Pflanzen, die hierbei in einer mehrteiligen Probennahme-Vorrichtung herangezogen werden. Die Probennahme-Vorrichtung umfasst ein Unterteil mit Anzuchtbehältern, in denen die Pflanzen während einer Anzuchtphase herangezogen werden. Weiter ist ein zu dem Unterteil im Wesentlichen korrespondierend geformtes Oberteil mit Probenbehältern umfasst, welches auf das Unterteil aufgesetzt wird. Nachdem Pflanzenspitzen der Pflanzen aus dem Unterteil in das aufgesetzte Oberteil hineingewachsen sind, kann zwischen das Oberteil und das Unterteil ein plattenförmiges Messer mit einer einzigen Klinge über eine Seitenlänge des Oberteils beziehungsweise Unterteils eingeschoben werden, welches die Pflanzenspitzen abschneidet. Gleichwohl besteht hier beim Durchtrennen der Pflanzen mit dem Messer die Gefahr einer Kreuzkontamination, da die Pflanzenspitzen beim Durchtrennen durch die Schwerkraft auf das Messer fallen und beim Einschub des Messers in Probenbehälter mit anderen Pflanzenspitzen geschoben werden können. Demzufolge ist in einer anschließenden Analysephase eine fehlerhafte Zuordnung der Analyseergebnisse zu einer Pflanze möglich.

Ebenfalls ist im Rahmen der sogenannten Ice-Cap-Methode die Verwendung einer mehrteiligen Probennahme-Vorrichtung für ein gattungsgemäßes Anzucht- und Probennahme-Verfahren bekannt. Hierbei werden in einem Oberteil der Probennahme-Vorrichtung Pflanzen aus einem Saatgut angezogen. Im Rahmen der Anzucht wachsen Teile der Wurzeln bis in den Bereich eines Unterteils der Probennahme-Vorrichtung. Um in Vorbereitung einer labortechnischen Untersuchung der Pflanzen die in das Unterteil gewachsenen Wurzelteile vom Rest der Pflanze zu trennen, wird das Unterteil der Probennahme-Vorrichtung mit den darin vorgesehenen Wurzelteilen geflutet und anschließend die Probennahme-Vorrichtung eingefroren. Nach dem Einfrieren kann das Unterteil vom Oberteil getrennt werden. Die im Unterteil festgefrorenen Wurzelteile werden abgerissen und nach dem Auftauen untersucht. Nachteilig ist hierbei, dass die Pflanzen durch das Einfrieren einem erhöhten Stress ausgesetzt sind und insbesondere nicht gewährleistet ist, dass der im Oberteil der Probennahme-Vorrichtung verbleibende, einmal eingefrorene Oberteil der Pflanzen mit dem Wurzelrest vital erhalten bleibt. Es kann insofern gegebenenfalls nicht für die Weiterentwicklung der Pflanze verwendet werden. Darüber hinaus ist der Aufwand zum Einfrieren der Probennahme-Vorrichtung vorrichtungsseitig relativ groß und die Durchführung des Verfahrens zeitaufwendig.

Die US 2013/0180171 A1 offenbart ein gattungsgemäßes Anzucht- und Probennahme-Verfahren für Pflanzen mit einer mehrteiligen Probennahme-Vorrichtung. Hierbei werden die Pflanzen in Anzuchtbehältern eines Oberteils herangezogen und bilden Wurzeln aus, die in korrespondierende und darunterliegende Probenbehälter eines Unterteils hineinwachsen. Für ein Durchtrennen der in die Probenbehälter hineingewachsenen Wurzeln muss hierbei das Oberteil gegenüber dem Unterteil angehoben werden. Erst im Anschluss kann zwischen das Oberteil und das Unterteil ein Messer oder dergleichen durchgeführt werden, das die Pflanzen durchtrennt. Ein gleichmäßiger Schnitt durch alle pflanzen ist hierbei nur möglich, wenn das Oberteil exakt parallel zu dem Unterteil angehoben ist. Zudem ist die Gefahr gegeben, dass beim Anheben des Oberteils die Pflanzen ungleichmäßig auseinandergerissen oder aus den Probenbehältern herausgerissen werden können. Demzufolge besteht auch hier ein nicht unerhebliches Risiko, dass Proben verschiedener Pflanzen durchmischt werden und eine fehlerhafte Zuordnung der Analyseergebnisse zu einer Pflanze möglich ist. Aufgabe der vorliegenden Erfindung ist es, ein verbessertes Anzucht- und Probennahme-Verfahren für Pflanzen anzugeben.

Die WO 2012/096568 A1 ist ein weiteres Beispiel für ein Anzucht- und Probennahme-Verfahren für Pflanzen.

Aufgabe der vorliegenden Erfindung ist es, ein verbessertes Anzucht- und Probennahme-Verfahren für Pflanzen anzugeben.

Zur Lösung der Aufgabe weist das erfindungsgemäße Anzucht- und Probennahme-Verfahren die Merkmale des Patentanspruchs 1 auf.

Nach dem erfindungsgemäßen Anzucht- und Probennahme-Verfahren werden die Pflanzen in einer mehrteiligen Probennahme-Vorrichtung herangezogen, die zum einen ein Oberteil mit einer Oberteilkennzeichnung und einer Anzahl von Anzuchtbehältern und zum anderen ein Unterteil mit einer Unterteilkennzeichnung und einer Anzahl von Probenbehältern vorsieht. Das Oberteil wird in einer Zusammenbauposition der Probennahme-Vorrichtung derart mit dem Unterteil verbunden, dass die Probenbehälter korrespondierend zu den Anzuchtbehältern unter ebendiesen angeordnet sind. Während einer Anzuchtphase, in der sich die Probennahme-Vorrichtung in der Zusammenbauposition befindet, werden die Pflanzen in den Anzuchtbehältern herangezogen. Die Anzuchtbehälter sind hierbei mit einem Substrat und/oder Nährstoffen gefüllt. Nachdem die Wurzeln der Pflanzen durch eine an jedem Anzuchtbehälter vorgesehene Bodenöffnung bis in den unterhalb des Anzuchtbehälters vorgesehenen Probenbehälter gewachsen sind, werden die in dem Unterteil vorgesehenen Wurzelteile mit einem Messer von der Pflanze getrennt. Dann wird in einer Analysephase das Unterteil der Probennahme-Vorrichtung mit den darin befindlichen Wurzelteilen einer Analyseeinrichtung zugeführt. Es wird die Unterteilkennzeichung der Probennahme-Vorrichtung erfasst und für die verschiedenen Wurzelteile wird eine phänotypische Beschreibung und/oder molekularbiologische Untersuchung durchgeführt. Dann wird in einer Selektionsphase das Oberteil der Probennahme-Vorrichtung mit den darin befindlichen Pflanzen einer Selektionseinrichtung zugeführt. Das Oberteil wird positioniert und die Oberteilkennzeichnung wird erfasst.

Vorteilhaft wird durch das erfindungsgemäße Anzucht- und Probennahme-Verfahren die phänotypische Beschreibung und/oder die molekularbiologische Untersuchung der Pflanzen signifikant vereinfacht. Es ist insbesondere so, dass eine manuelle, einzelne Probennahme an den Pflanzenblättern mittels einer Zange und/oder einem Stanzwerkzeug vermieden und stattdessen eine molekularbiologische Untersuchung der Wurzelteile vorgenommen wird. Die Wurzelteile können dabei für eine Vielzahl von Pflanzen gemeinsam abgeschnitten werden. Sie sind unmittelbar in den Probenbehältern des Unterteils vorgesehen und können ohne weitere manuelle Eingriffe der Analyse zugeführt werden.

Das erfindungsgemäße Verfahren ist überdies sehr schonend. Es wird beispielsweise darauf verzichtet, die Probennahme-Vorrichtung mit den Pflanzen zu fluten beziehungsweise einzufrieren. Insofern bleiben die Pflanzen im Oberteil vital erhalten und sie können in sehr einfacher Weise für die weitere Züchtung verwendet werden. Darüber hinaus kann die Probennahme sehr schnell durchgeführt werden.

Zusätzlich ist einer Fehleranfälligkeit durch die Reduzierung der manuellen Arbeitsschritte und insbesondere durch einen Verzicht auf die manuelle Probennahme und das manuelle Einbringen der Proben in die Probenbehälter vorgebeugt.

Aus Gründen der Übersicht, Verständlichkeit und Einfachheit wird im Rahmen der folgenden Beschreibung für die Pflanze als Ganzes, das heißt die Pflanze mit den in das Unterteil gewachsenen Wurzeln, und für den nach dem Durchtrennen der Wurzeln im Oberteil der Probennahme-Vorrichtung verbleibenden vitalen Rest der Pflanze einheitlich der Begriff "Pflanze" verwendet.

Nach der Erfindung werden die in das Unterteil gewachsenen Wurzelteile der Pflanzen mittels eines als Teil der Probennahme-Vorrichtung ausgebildeten und in der Zusammenbauposition an dem Unterteil und/oder dem Oberteil gehaltenen Messers von den Pflanzen getrennt. Insbesondere kann vorgesehen sein, dass die Wurzelteile von den Pflanzen getrennt werden, indem das Messer entlang einer an dem Unterteil festgelegten Schneidplatte der Probennahme-Vorrichtung geführt ist. Vorteilhaft kann das Trennen der Wurzelteile vereinfacht und das Anzucht- und Probennahme-Verfahren weiter beschleunigt werden, indem das Messer als Teil der Probennahme-Vorrichtung realisiert ist. Zudem ergibt sich durch das Integrieren des Messers und/oder das Vorsehen der Schneidplatte eine stets gleiche, exakte Trennposition mit der Folge, dass die Ausgangsbedingungen für die anschließende Analyse stets gleich und in einem hohen Maße reproduzierbar sind. Zu diesem Zweck kann insbesondere vorgesehen sein, dass das Messer und/oder die Schneidplatte, welche jeweils als Teil der Probennahme-Vorrichtung ausgeführt sind, zwischen dem Oberteil und dem Unterteil der Probennahme-Vorrichtung positioniert sind.

Nach einer besonders vorteilhaften Weiterbildung der Erfindung werden in der Zusammenbauposition der Probennahme-Vorrichtung den Anzuchtbehältern mit den Bodenöffnungen zugewandte Probenbehälteröffnungen der Probenbehälter von der Schneidplatte überdeckt. Die Schneidplatte sieht hierbei eine Anzahl von Schneidlöchern als Durchgangsöffnungen für die Wurzeln der Pflanzen vor, welche korrespondierend zu der Lage der Bodenöffnungen und der Probenbehälteröffnungen angeordnet sind. Vorteilhaft wirkt eine Überdeckung der Probenbehälteröffnungen durch die Schneidplatte einer unerwünschten Kontamination der Proben wirksam vor. Es wird insofern durch die Schneidplatte verhindert, dass die Wurzeln einer Pflanze in einem anderen als den unterhalb des zugeordneten Anzuchtbehälters vorgesehenen Probenbehälter wachsen. Der Schneidplatte kommt im Rahmen des erfindungsgemäßen Anzucht- und Probennahme-Verfahrens insofern eine doppelte Bedeutung zu. Sie dient zum einen dem Führen des Messers und gewährleistet insofern eine stets gleiche Probennahme beziehungsweise das Durchtrennen der Wurzeln an einer definierten Stelle. Zum anderen dient sie zur Verbesserung der Analyse, indem sie einer Kontamination entgegenwirkt.

Nach einer Weiterbildung der Erfindung ist das zwischen dem Oberteil und dem Unterteil der Probennahme-Vorrichtung vorgesehene Messer nach Art einer MesserLochplatte mit einer Anzahl von Schneidlöchern ausgebildet, welche als Durchgangsöffnungen ausgebildet sind und während der Anzuchtphase korrespondierend zu den Durchgangsöffnungen der Schneidplatte so angeordnet sind, dass die Wurzeln in den Probenbehältern wachsen können. Zum Durchtrennen der Wurzeln wird das Messer um einen vorbestimmten Hub entlang der Schneidplatte geführt. Der Hub ist so gewählt, dass zum einen die Wurzeln zuverlässig durchtrennt werden und dass zum anderen jede an der Lochplatte des Messers ausgebildete Durchgangsöffnung nur mit einer Durchgangsöffnung der Schneidplatte zusammenwirkt. Insofern ist der Hub größer gewählt als ein Durchmesser der an der Schneidplatte und dem Messer vorgesehenen Durchgangöffnungen und kleiner gewählt als ein Abstand von zwei in die Hubrichtung benachbarten Probenbehältern. Auf diese Weise ist einer unerwünschten Durchmischung (Kontamination) der Proben wirkungsvoll entgegengewirkt.

Nach einer Weiterbildung der Erfindung wird nach dem Durchtrennen der Wurzeln zur Vorbereitung der Analyse das Unterteil der Probennahme-Vorrichtung mit den darin vorgesehenen Wurzelteilen zusammen mit der Schneidplatte von dem Oberteil der Probennahme-Vorrichtung getrennt.

Nach einer Weiterbildung der Erfindung wird dann das Unterteil mit den darin vorgesehenen Wurzelteilen entwässert. Die Entwässerung kann beispielsweise durchgeführt werden, indem das Unterteil zusammen mit der Schneidplatte in eine Zentrifugiereinrichtung eingesetzt wird und das Wasser unter Einwirkung der Zentrifugalkraft über die an der Schneidplatte vorgesehenen Durchgangsöffnungen herausgeschleudert wird beziehungsweise austritt. Die Wurzelteile verbleiben dabei aufgrund ihrer stofflichen Konsistenz und der geringen Größe der Durchgangsöffnungen in den Probenbehältern des Unterteils. Vorteilhaft wird die weitergehende Untersuchung der Wurzelteile durch das Entfernen des Wassers begünstigt. Die Entwässerung kann bei Nutzung der Zentrifugiereinrichtung schnell und einfach realisiert werden. Beispielsweise kann in einem weiteren Verfahrensschritt vorgesehen werden, dass die in dem Unterteil vorgesehenen Wurzelteile mittels der Zentrifugalkraft an einen Grund der Probenbehälter verbracht werden. Zu diesem Zweck kann das Unterteil mit der Schneidplatte und den in dem Unterteil vorgesehenen Wurzelteilen in der Zentrifugiereinrichtung gedreht werden und/oder es kann die Drehrichtung der Zentrifugiereinrichtung geändert werden.

Nach der Erfindung wird das Unterteil mit den darin vorgesehenen Wurzelteilen und der Schneidplatte nach dem Durchtrennen der Wurzeln und/oder dem Entwässern einer Stanzeinrichtung zugeführt. Mittels eines Stanzstempels der Stanzeinrichtung werden dann rund um die Schneidlöcher ringförmige, bevorzugt kreisringförmige Abschnitte aus der Stanzplatte herausgestanzt und in das Innere des jeweils zugeordneten Probenbehälters überführt. Vorteilhaft kann durch das Stanzen der ringförmigen Abschnitte das Risiko einer Kontamination der Proben weiter reduziert werden. Insbesondere werden die abgetrennten Wurzelteile der Pflanzen, welche sich nach dem Durchtrennen noch teilweise in den Durchgangsöffnungen der Schneidplatte befinden, zusammen mit dem Abschnitt in den Probenbehälter überführt. Nach dem Stanzen kann also die Schneidplatte von dem Unterteil entfernt werden, ohne dass das Risiko besteht, dass beim Entfernen der Schneidplatte an ihr anhaftende Wurzelteile aus den Probenbehältern gezogen werden beziehungsweise es zu einer Kontamination der Proben kommt. Die Schneidplatte der Probennahme-Vorrichtung wird insbesondere von dem Unterteil entfernt, bevor das Unterteil der Analyseeinrichtung zugeführt wird zur Durchführung der phänotypischen Beschreibung und/oder der molekularbiologischen Untersuchung der Proben.

Nach einer Weiterbildung der Erfindung wird vor dem Herausstanzen der Abschnitte aus der Schneidplatte ein an einem der Schneidplatte zugewandten freien Ende an den Stanzstempeln vorgesehener Positionierkopf in Eingriff gebracht mit den Schneidlöchern der Schneidplatte. Vorteilhaft wird durch das Vorsehen der Positionierköpfe und das Eingreifen derselben in die Schneidlöcher der Schneidplatte eine Lagezuordnung der Schneidplatte mit dem Unterteil zu der Stanzeinrichtung der Probenbehälter bewirkt sowie einer Durchmischung beziehungsweise Kontamination der Proben ist hierdurch vorgebeugt.

Nach einer Weiterbildung der Erfindung wird ein Anzuchtbehälter des in der Selektionseinrichtung positionierten Oberteils der Probennahme-Vorrichtung mittels eines Signalgebers der Selektionseinrichtung gekennzeichnet. Teile der Pflanze oder die gesamte Pflanze aus dem gekennzeichneten Anzuchtbehälter können dann für die weitere Züchtung beziehungsweise Behandlung entnommen werden. Vorteilhaft kann durch die Kennzeichnung des Anzuchtbehälters einer fehlerhaften Entnahme entgegengewirkt werden. Das erfindungsgemäße Anzucht- und Probennahme-Verfahren kann insofern sehr zuverlässig durchgeführt werden.

Nach einer alternativen Ausführungsform der Erfindung kann die Entnahme der selektierten Pflanzen beziehungsweise eines Teils derselben automatisiert beziehungsweise teilautomatisiert erfolgen mittels eines Greifers der Selektionseinrichtung, welcher zunächst zu einem ausgesuchten Anzuchtbehälter positioniert wird und dann die Entnahme für ebendiesen Anzuchtbehälter durchführt. Vorteilhaft kann durch die Automatisierung die Selektion beschleunigt werden. Darüber hinaus ist einer fehlerhaften Entnahme vorgebeugt.

Nach einer Weiterbildung der Erfindung wird in der Selektionsphase eine Mehrzahl von Anzuchtbehältern eines gleichen Oberteils der Probennahme-Vorrichtung sequenziell mittels des Signalgebers der Selektionseinrichtung gekennzeichnet. Vorteilhaft kann durch das sequenzielle Kennzeichnen der Anzuchtbehälter eines gleichen Oberteils einer Verwechslung der Proben wirksam vorgebeugt werden. Ein technischer Mitarbeiter kann sich insofern zu jedem Zeitpunkt auf einen einzigen Anzuchtbehälter beziehungsweise eine einzige darin vorgesehene Pflanze konzentrieren.

Nach einer Weiterbildung der Erfindung wird die Probennahme-Vorrichtung während der Anzuchtphase mittels Leuchtdioden beleuchtet. Es hat sich hier gezeigt, dass durch das Beleuchten der Pflanzen während der Anzuchtphase das Wachstum der Wurzeln beschleunigt werden kann, während zugleich ein Längenwachstum des Sprosses gehemmt wird. Hierbei spielen die Lichtintensität und die Zusammensetzung des Lichtspektrums eine Rolle. Beispielsweise wird sichtbares Licht im

Wellenlängenbereich von 400 nm bis 700 nm verwendet. Eine Beleuchtungseinrichtung ist dabei insbesondere so ausgebildet, dass das Licht beispielsweise vollspektral, das heißt über den gesamten Wellenlängenbereich ausgestrahlt wird und/oder dass der Blaubereich (zirka 400 nm bis 500 nm), der Grün-Gelbbereich (zirka 500 nm bis 600 nm) und der Rotbereich (zirka 600 nm bis 700 nm) getrennt angesteuert und/oder gedimmt werden können. Speziell kann vorgesehen sein, dass für unterschiedliche Kulturarten individuell angepasste Beleuchtungsparameter verwendet werden und/oder dass jeweils einzelne Teilbereich von definierter Größe und Form unter Berücksichtigung von Wandreflektionen und/oder Überlagerungseffekten homogen und mit einer definierten Beleuchtungsstärke beziehungsweise Spektralzusammensetzung ausgeleuchtet werden.

Die Beleuchtungseinrichtung kann Mittel zum Kühlen der Leuchtdioden vorsehen. Beispielsweise kann eine aktive Kühlung, insbesondere ein Wasser- beziehungsweise Flüssigkeitskühlung vorgesehen werden, um die Abwärme schnell und kontrolliert abführen zu können. Alternativ kann eine passive Konvektionskühlung für die Leuchtdioden vorgesehen werden. Beispielsweise kann mittels der Abwärme die Anzuchtumgebung erwärmt beziehungsweise kontrolliert temperiert werden. Insbesondere können die Regalböden, auf denen eine Vielzahl von Probennahme-Vorrichtungen während der Anzucht aufgestellt ist, definiert temperiert werden.

Nach einer Weiterbildung der Erfindung sind die Anzuchtbehälter mit einem Granulat, beispielsweise Ziegelgrus, als Substrat und/oder Nährstoffen gefüllt. Die Anzuchtbehälter werden während der Anzuchtphase von oben bewässert. Zu diesem Zweck werden beispielsweise Sprühdüsen verwendet, die zur Bewässerung computergesteuert über die Pflanzen bewegt werden. Beispielsweise werden die Sprühdüsen nach der Bewässerung so außer Eingriff verbracht und positioniert, dass die Beleuchtung der Pflanzen ohne Beschattung erfolgen kann. Vorteilhaft kann durch die Verwendung von Ziegelgrus und die Bewässerung der Anzuchtbehälter von oben der Pflegeaufwand während der Anzuchtphase reduziert werden. Zudem kann anders als bei der Ice-Cap-Methode darauf verzichtet werden, die Probennahme-Vorrichtung in ein Wasserbecken zu stellen, dies jedenfalls teilweise zu fluten und mittels einer Pumpe in dem Wasserbecken einen konstanten Wasserpegel zu realisieren. Es vereinfacht sich also das Anzucht- und Probennahme-Verfahren sowohl in Bezug auf die Handhabung als auch in Bezug auf die vorzuhaltenden Vorrichtungen signifikant. Nach einer Weiterbildung der Erfindung erfolgt die Aussaat signalunterstützt derart, dass mittels einer Aussaateinrichtung ein auf einer Umverpackung eines Saatguts der Pflanzen vorgesehene Saatgutkennzeichnung eingelesen und mittels eines Signalgebers der Aussaateinrichtung ein ausgewählter Anzuchtbehälter der Probennahme-Vorrichtung gekennzeichnet wird zur Durchführung der Aussaat. Vorteilhaft vereinfacht sich das Anzucht- und Probennahme-Verfahren weiter, da bereits bei der Aussaat eine Entsprechung zwischen einer Pflanze beziehungsweise dem Saatgut dieser Pflanze einerseits und ein dem Anzuchtbehälter der Probennahme-Vorrichtung andererseits hergestellt ist. Verwechslungen, beispielsweise eine fehlerhafte Notierung des Anzuchtbehälters beziehungsweise des Saatguts und/oder eine falsche Zuordnung derselben, wird vermieden.

Nach einer alternativen Ausführungsform der Erfindung kann die Aussaat maschinell unterstützt erfolgen, indem das Saatgut mittels eines Greifers der Aussaateinrichtung aufgenommen und nach dem Positionieren des Greifers in einem ausgewählten Anzuchtbehälter der Probennahme-Vorrichtung vorgesehen wird. Vorteilhaft kann hierdurch das erfindungsgemäße Anzucht- und Probennahme-Verfahren weiter beschleunigt werden. Zudem ist durch die Automatisierung Fehlern und Verwechslungen entgegengewirkt.

Nach einer Weiterbildung der Erfindung werden die Analyseeinrichtung und/oder eine das Messer der Probennahme-Vorrichtung betätigende Trenneinrichtung und/oder die Selektionseinrichtung und/oder die Aussaateinrichtung und/oder die Stanzeinrichtung steuerungstechnisch und/oder datentechnisch derart gekoppelt, dass die Oberteilkennzeichnung und/oder die Unterteilkennzeichnung und/oder die Saatgutkennzeichnung erfasst werden und mit den Analyseergebnissen der molekularbiologischen Untersuchung und/oder der phänotypischen Beschreibung der Wurzelteile verknüpft werden. Die Ergebnisse, die Kennzeichnungen und die Zuordnung können beispielsweise in einer Datenbank abgespeichert werden. Beispielsweise sind zu diesem Zweck die Oberteilkennzeichnung und/oder die Unterteilkennzeichnung und/oder die Saatgutkennzeichnung nach Art eines Barcodes oder dergleichen ausgebildet und Mittel zum optischen Einlesen der Kennzeichnungen vorgesehen. In Bezug auf die verschiedenen Anzucht- und Probenbehälter der Probennahme-Vorrichtung kann die Identifikation beispielsweise bei einer matrixartigen Anordnung gewährleistet werden, indem die Reihe und die Spalte der jeweiligen Behälter erfasst ist. Vorteilhaft erfolgt insofern eine eindeutige Zuordnung der Wurzelteile zu den Probenbehältern und den zugeordneten Anzuchtbehältern, sodass einer Verwechslung der Proben beziehungsweise Pflanzen besonders wirkungsvoll vorgebeugt ist beziehungsweise deren Identifikation zuverlässig erfolgen kann.

Nach einer Weiterbildung der Erfindung verwenden die Aussaateinrichtung und die Selektionseinrichtung einen gemeinsamen Signalgeber und/oder Greifer. Beispielsweise kennzeichnet der Signalgeber der Selektionseinrichtung beziehungsweise der Aussaateinrichtung den Anzuchtbehälter des Oberteils beziehungsweise den Probenbehälter des Unterteils optisch. Es kann als Signalgeber insofern beispielsweise eine Leuchtdiode oder ein Leuchtdioden-Array vorgesehen sein, bei dem die Anzahl und/oder Anordnung der Leuchtdioden an die Anzahl und Anordnung der Anzuchtbehälter des Oberteils der Probennahme-Vorrichtung angepasst ist.

Nach einer Weiterbildung der Erfindung werden die Probenbehälter des Unterteils in einem 96er Deepwellplatten-Format ausgebildet. Vorteilhaft reduziert sich durch die Verwendung des genormten 96er Deepwellplatten-Formats der Flächenaufwand für die Anzucht der Pflanzen etwa um 90 % mit der Folge, dass beispielsweise in einer gleichen Gewächshausfläche signifikant mehr Pflanzen herangezogen beziehungsweise die gleiche Anzahl von Pflanzen in einem deutlich kleineren und günstiger zu bewirtschaftenden Gewächshaus herangezogen werden kann.

Aus den weiteren Unteransprüchen und der nachfolgenden Beschreibung sind weitere Vorteile, Merkmale und Einzelheiten der Erfindung zu entnehmen. Dort erwähnte Merkmale können jeweils einzeln für sich oder auch in beliebiger Kombination erfindungswesentlich sein. Die Zeichnungen dienen lediglich beispielhaft der Klarstellung der Erfindung und haben keinen einschränkenden Charakter.

Es zeigen:
- Fig. 1: ein erstes Ausführungsbeispiel einer zur Durchführung des erfindungsgemäßen Anzucht- und Probennahme-Verfahrens verwendeten Probennahme-Vorrichtung in einer ersten perspektivischen Explosionsdarstellung;
- Fig. 2: das erste Ausführungsbeispiel der Probennahme-Vorrichtung in einer zweiten perspektivischen Explosionsdarstellung;
- Fig. 3: das erste Ausführungsbeispiel der Probennahme-Vorrichtung in einer dritten perspektivischen Explosionsdarstellung;
- Fig. 4: das erste Ausführungsbeispiel der Probennahme-Vorrichtung in einer ersten perspektivischen Zusammenbaudarstellung;
- Fig. 5: das erste Ausführungsbeispiel der Probennahme-Vorrichtung in einer zweiten perspektivischen Zusammenbaudarstellung;
- Fig. 6: das erste Ausführungsbeispiel der Probennahme-Vorrichtung in einer Draufsicht;
- Fig. 7: ein zweites Ausführungsbeispiel der Probennahme-Vorrichtung in einer zur Fig. 4 korrespondierenden perspektivischen Zusammenbaudarstellung;
- Fig. 8: ein drittes Ausführungsbeispiel der Probennahme-Vorrichtung in einer ersten perspektivischen Explosionsdarstellung in teilweiser Ansicht;
- Fig. 9: das dritte Ausführungsbeispiel der Probennahme-Vorrichtung in einer zweiten perspektivischen Explosionsdarstellung in teilweiser Ansicht;
- Fig. 10: das dritte Ausführungsbeispiel der Probennahme-Vorrichtung in einer Detailansicht im Bereich einer Schneidplatte in einer geschnittenen, teilweisen Ansicht;
- Fig. 11: das dritte Ausführungsbeispiel der Probennahme-Vorrichtung in einer ersten perspektivischen Eingriffsdarstellung in einer teilweisen Ansicht,
- Fig. 12: ein viertes Ausführungsbeispiel der Probennahme-Vorrichtung in einer ersten perspektivischen Explosionsdarstellung in teilweiser Ansicht,
- Fig. 13: ein fünftes Ausführungsbeispiel der Probennahme-Vorrichtung in einer perspektivischen Explosionsdarstellung in einer teilweisen Ansicht,
- Fig. 14: das fünfte Ausführungsbeispiel der Probennahme-Vorrichtung in einer Draufsicht in teilweiser Ansicht,
- Fig. 15: eine bei der Durchführung des erfindungsgemäßen Anzucht- und Probennahme-Verfahrens verwendete Trenneinrichtung in einer Grundposition in einer perspektivischen Darstellung,
- Fig. 16: die Trenneinrichtung nach Fig. 15 mit der darin eingesetzten Probennahme-Vorrichtung in der Grundposition in der gleichen perspektivischen Darstellung,
- Fig. 17: die Trenneinrichtung mit der Probennahme-Vorrichtung in einer Betätigungsposition,
- Fig. 18: eine bei der Durchführung des erfindungsgemäßen Anzucht- und Probennahme-Verfahrens verwendete Stanzeinrichtung in einer Grundposition,
- Fig. 19: die Stanzeinrichtung nach Fig. 18 in einer Betätigungsposition,
- Fig. 20: einen ersten Schritt eines Stanzvorgangs in einer Prinzipdarstellung im Schnitt,
- Fig. 21: einen zweiten Schritt des Stanzvorgangs der gleichen Prinzipdarstellung im Schnitt,
- Fig. 22: einen dritten Schritt des Stanzvorgangs der gleichen Prinzipdarstellung im Schnitt,
- Fig. 23: einen vierten Schritt des Stanzvorgangs der gleichen Prinzipdarstellung im Schnitt,
- Fig. 24: einen fünften Schritt des Stanzvorgangs der gleichen Prinzipdarstellung im Schnitt,
- Fig. 25: einen Signalgeber einer zur Durchführung des erfindungsgemäßen Anzucht- und Probennahme-Verfahrens verwendeten Selektionseinrichtung und/oder Aussaateinrichtung in einer ersten perspektivischen Darstellung und

Fig. 26 den Signalgeber nach Fig. 25 in einer zweiten perspektivischen Darstellung.

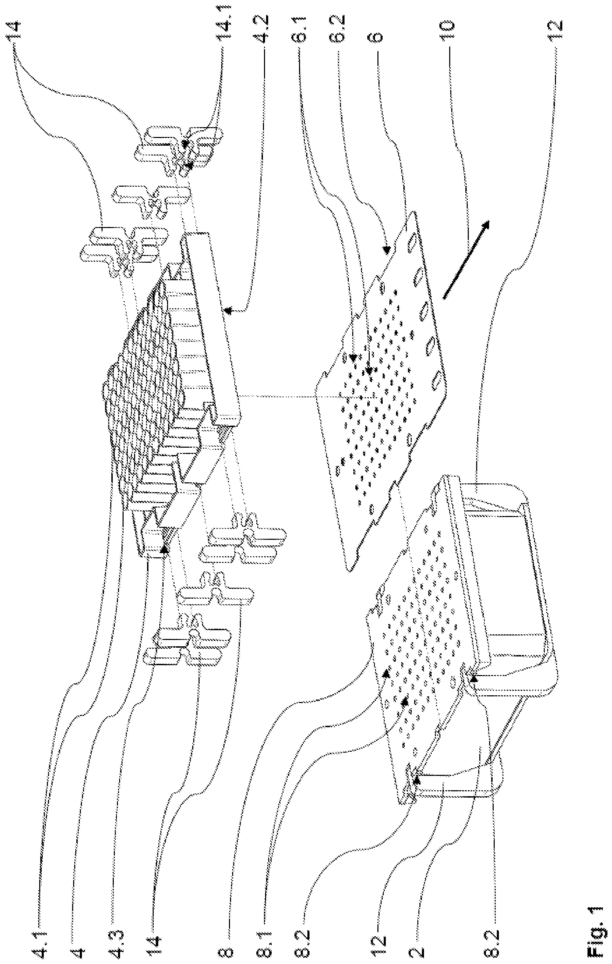

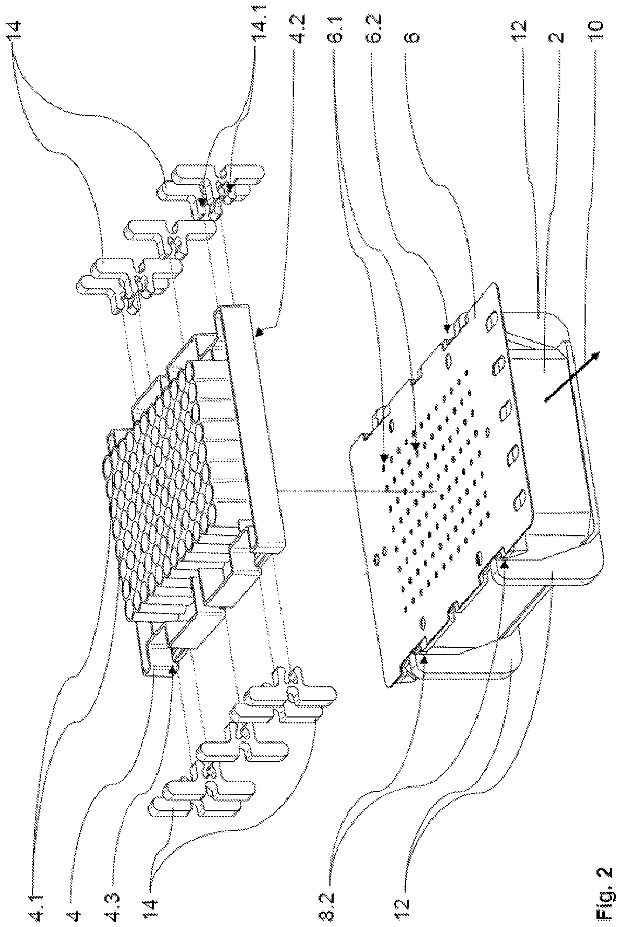

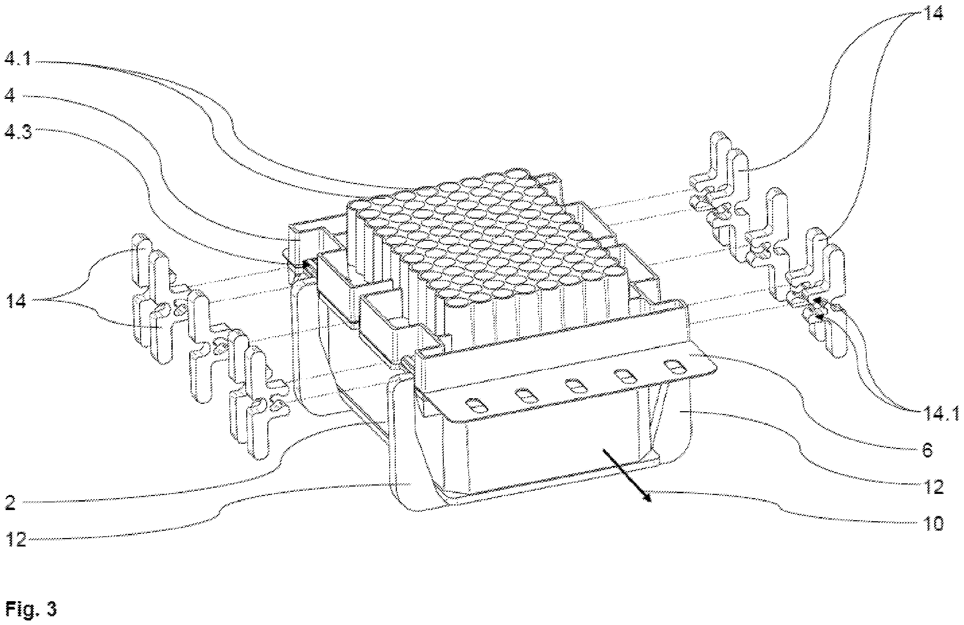

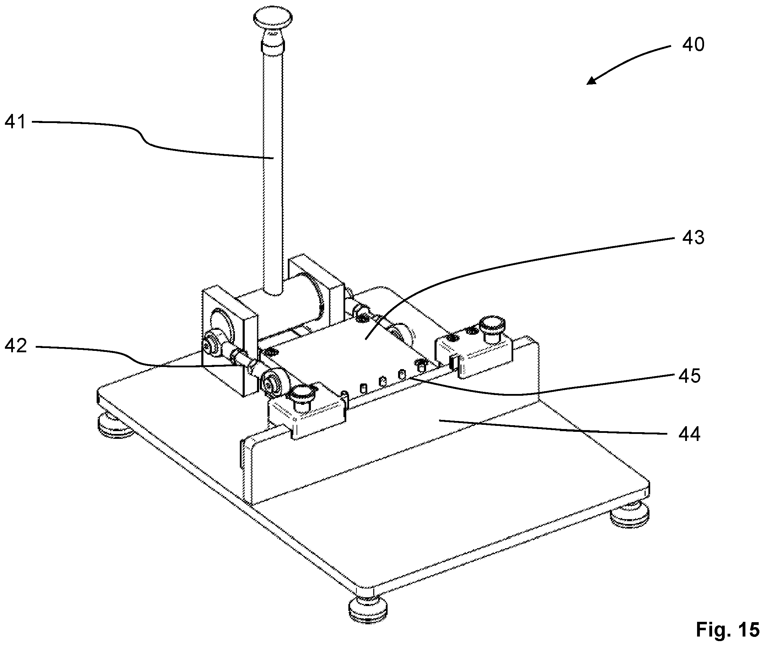

Die Fig. 1 bis 14 zeigen voneinander verschiedene Ausführungsbeispiele einer zur Durchführung des erfindungsgemäßen Anzucht- und Probennahme-Verfahrens verwendeten Probennahme-Vorrichtung. Weitere zur Durchführung des erfindungsgemäßen Anzucht- und Probennahme-Verfahrens verwendbare Komponenten und Funktionseinrichtungen sowie Detaildarstellungen hierzu sind in den Fig. 15 bis 26 gezeigt.

Gleiche oder gleichwirkende Bauteile sind einheitlich mit gleichen Bezugszeichen bezeichnet. Es werden lediglich die Unterscheidungsmerkmale der auf das erste Ausführungsbeispiel der Probennahme-Vorrichtung folgenden Ausführungsbeispiele zu dem ersten Ausführungsbeispiel erläutert. Im Übrigen stimmen die Ausführungsbeispiele überein.

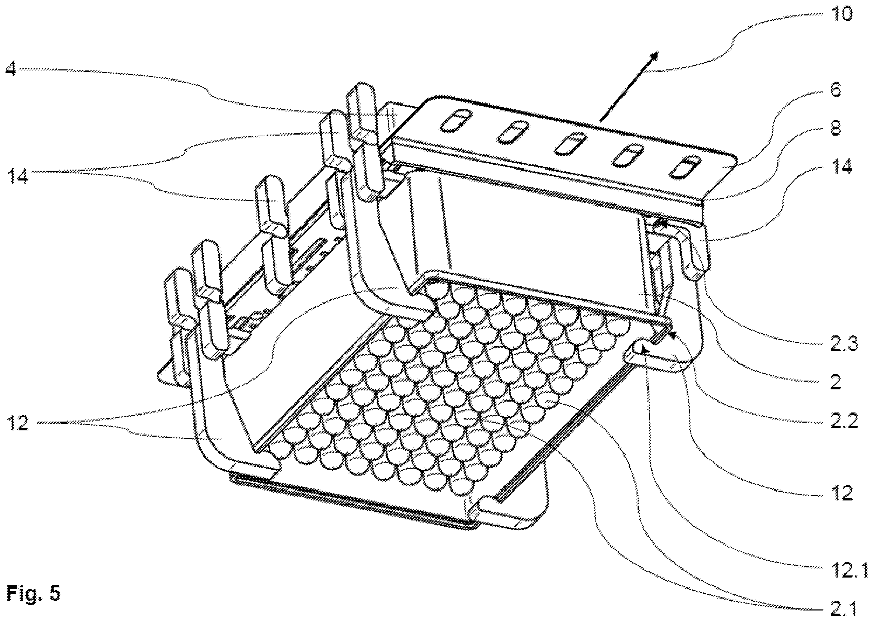

Fig. 1 zeigt ein erstes Ausführungsbeispiel einer Probennahme-Vorrichtung in einer perspektivischen Explosionsdarstellung. Die Probennahme-Vorrichtung weist ein bevorzugt jedenfalls in Teilen aus Kunststoff gebildetes Unterteil 2 mit einer Mehrzahl von Probenbehältern 2.1 auf, die besonders deutlich in einer perspektivischen Unterseitenansicht der Probennahme-Vorrichtung nach Fig. 5 erkennbar sind. Die Probenbehälter 2.1 sind vorliegend im 96er Deepwellplatten-Format realisiert und nach Art einer 12x8-Matrix angeordnet.

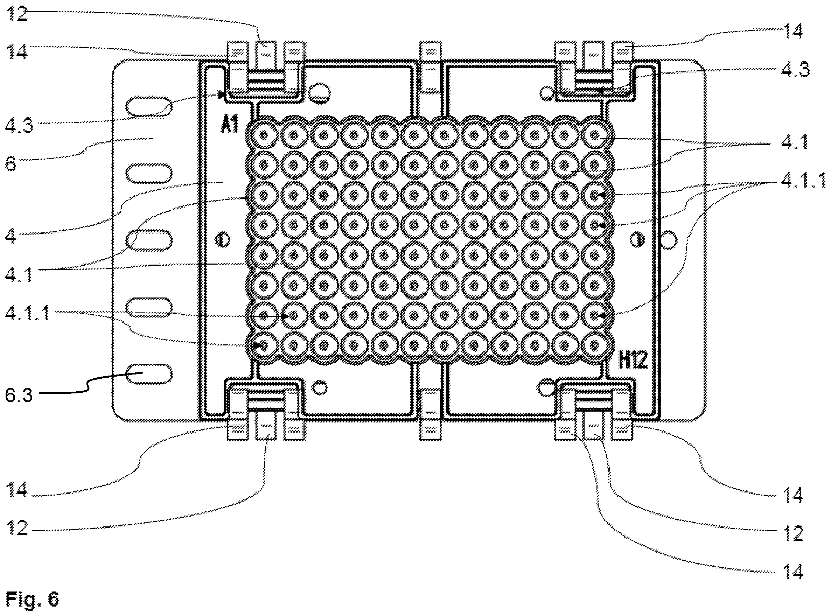

Ferner weist die Probennahme-Vorrichtung ein aus Kunststoff hergestelltes Oberteil 4 mit einer Mehrzahl von Anzuchtbehältern 4.1, ein bevorzugt aus Kunststoff oder Metall hergestelltes Messer 6 und eine bevorzugt aus Kunststoff hergestellte Schneidplatte 8 auf. In jedem Anzuchtbehälter 4.1 ist jeweils eine Bodenöffnung 4.1.1 ausgebildet, die in Fig. 6 zu erkennen sind. Die Probenbehälter 2.1 sind hier integraler Bestandteil des Unterteils 2 und die Anzuchtbehälter 4.1 sind integraler Bestandteil des Oberteils 4. Das Unterteil 2 und das Oberteil 4 sind in dem ersten Ausführungsbeispiel der Erfindung also jeweils für sich einstückig ausgebildet.

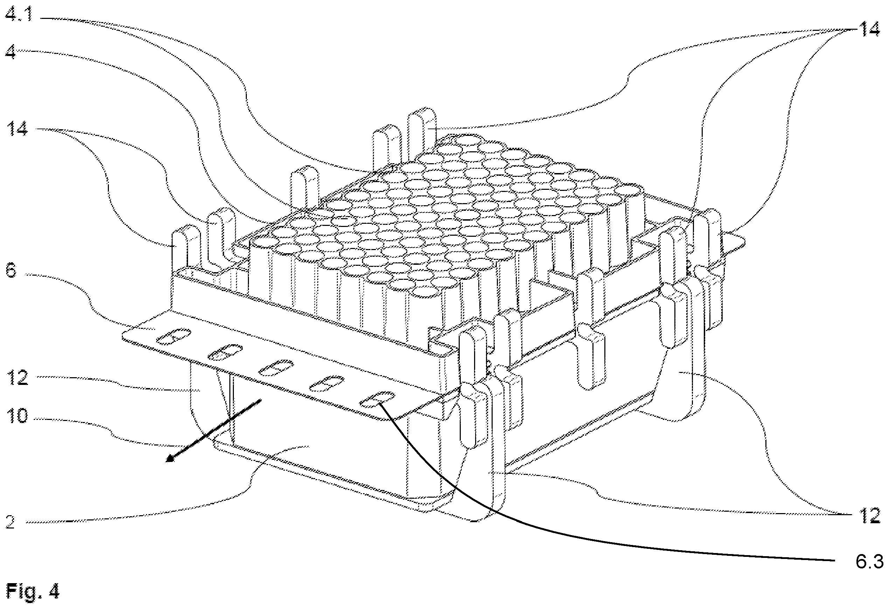

Wie aus der Zusammenschau der Figuren deutlich wird, ist jedem Anzuchtbehälter 4.1 genau ein Probenbehälter 2.1 und jeder Bodenöffnung 4.1.1 genau eine Probenbehälteröffnung in einer in den Fig. 4 bis 6 dargestellten Zusammenbauposition zugeordnet. Die Probenbehälteröffnungen sind nicht explizit dargestellt, da sie von der Schneidplatte 8 überdeckt sind.

Die eindeutige Zuordnung von genau einem Anzuchtbehälter 4.1 zu genau einem Probenbehälter 2.1 ist hier deshalb sinnvoll, weil die Probennahme-Vorrichtung insbesondere für die phänotypische Beschreibung und/oder die molekularbiologische Untersuchung von Pflanzen vorgesehen ist. Für den Erfolg derartiger Analysen ist es wichtig, dass es zu keiner ungewünschten Durchmischung (Kreuzkontamination) der einzelnen Gewebeproben (Genotypen) kommt. Dies ist durch die vorgenannte Ausbildung der Probennahme-Vorrichtung gewährleistet.

Die Anzuchtbehälter 4.1 wurden zunächst mit nicht dargestelltem Substrat und/oder Nährstoffen, zum Beispiel Ziegelgrus, für das Pflanzenwachstum befüllt. In den einzelnen Anzuchtbehältern 4.1 wird im Gebrauch der erfindungsgemäßen Probennahme-Vorrichtung je eine Pflanze gezogen. Die Wurzeln der nicht dargestellten Pflanzen wachsen im weiteren Verlauf der Pflanzenentwicklung durch das Substrat und die Bodenöffnungen 4.1.1 der Anzuchtbehälter 4.1 und die Probenbehälteröffnungen in die zu den einzelnen Anzuchtbehältern 4.1 korrespondierenden Probenbehälter 2.1 des Unterteils 2 hinein.

Das Messer 6 und die Schneidplatte 8 sind in der in den Fig. 4 bis 6 dargestellten Zusammenbauposition der erfindungsgemäßen Probennahme-Vorrichtung derart zwischen dem Oberteil 4 und dem Unterteil 2 angeordnet, dass die durch die Bodenöffnungen 4.1.1 der Anzuchtbehälter 4.1 heraus- und durch die Probenbehälteröffnungen in den jeweiligen Probenbehälter 2.1 hineinragenden Wurzeln mittels des Messers 6 und der Schneidplatte 8 durchtrennbar sind.

Das Messer 6 und die Schneidplatte 8 sind hier als eine Messerlochplatte 6 und eine Loch-Schneidplatte 8 ausgebildet. Dabei ist die Anzahl von in den beiden Lochplatten 6, 8 ausgebildeten Schneidlöchern 6.1, 8.1 identisch mit der Anzahl der Bodenöffnungen 4.1.1 der Anzuchtbehälter 4.1 und der Anzahl der Probenbehälteröffnungen der Probenbehälter 2.1. Die Anzuchtbehälter 4.1 und die Probenbehälter 2.1 sind bevorzugt aus Kunststoff gebildet.

In der in den Fig. 4 bis 6 dargestellten Zusammenbauposition sind die an dem Messer 6 und der Schneidplatte 8 ausgebildeten Schneidlöcher 6.1, 8.1 deckungsgleich mit den dazu korrespondierenden Bodenöffnungen 4.1.1 und Probebehälteröffnungen. Entsprechend können die Wurzeln der in den Anzuchtbehältern 4.1 gezogenen Pflanzen ungehindert von dem jeweiligen Anzuchtbehälter 4.1 durch die Bodenöffnungen 4.1.1, die Schneidlöcher 6.1, 8.1 und die Probenbehälteröffnungen in den jeweiligen Probenbehälter 2.1 hineinwachsen.

Zur Durchtrennung der nicht dargestellten Wurzeln wird das Messer 6 entlang der Schneidplatte 8 in Richtung des Pfeiles (Hubrichtung 10) geführt, so dass die Wurzeln zwischen dem Messer 6 und der Schneidplatte 8, also an den Kanten der Schneidlöcher 6.1, 8.1, abgeschert werden. Um die Bewegung des Messers 6 parallel in die Hubrichtung 10 zu ermöglichen, weist das Messer 6 an dessen beiden Längsseiten Längsausnehmungen 6.2 auf, was nachfolgend noch näher erläutert ist. Ferner sind langlochartig ausgebildete Aufnahmen 6.3 zum Betätigen des Messers 6 vorgesehen.

Die Schneidplatte 8 ist vorliegend als ein abnehmbarer Deckel 8 für das Unterteil 2 ausgebildet, wobei die Schneidplatte 8 mittels erster Klemmen 12 an dem Unterteil 2 lösbar befestigt ist. Wie insbesondere aus den Fig. 1 und 5 ersichtlich ist, umfassen die ersten Klemmen 12 das Unterteil 2 und die Schneidplatte 8 klammerartig und rasten mit an den freien Enden der ersten Klemmen 12 ausgebildeten Rastvorsprüngen 12.1 in dazu korrespondierend ausgebildete Rastaufnahmen 8.2 der Schneidplatte 8 und hinter einen an dem Unterteil 2 ausgebildeten Rastkragen 2.2 ein.

Damit die ersten Klemmen 12 die Bewegung des Messers 6 entlang der Schneidplatte 8 nicht behindern, überragt die Schneidplatte 8 die ersten Klemmen 12 in der in Zusammenbauposition von dem Unterteil 2 und der Schneidplatte 8.

Ferner ist die Baugruppe, die aus dem Unterteil 2 und der mittels der ersten Klemmen 12 daran befestigten Schneidplatte 8 gebildet ist, mit dem Messer 6 und dem Oberteil 4 durch zweite Klemmen 14 lösbar verbunden. Hierfür sind die vorgenannten Bauteile passgenau übereinandergelegt und mit den zweiten Klemmen 14 geklemmt. Analog zu der Klemmverbindung zwischen dem Unterteil 2 und der Schneidplatte 8 weisen das Unterteil 2 und das Oberteil 4 Rastaufnahmen 2.3 und 4.3 auf, die mit an freien Enden der zweiten Klemmen 14 ausgebildeten Rastvorsprüngen 14.1 in einer in den Fig. 4 bis 6 dargestellten Gebrauchslage eine lösbare Rastverbindung eingehen.

Zur besseren Führung des Messers 6 zwischen der an dem Unterteil 2 angeordneten Schneidplatte 8 und dem Oberteil 4 ist der dem Messer 6 zugewandte Boden 4.2 des Oberteils 4 als eine Messerführung 4.2 abschnittsweise eben beziehungsweise plan ausgebildet.

Die erfindungsgemäße Probennahme-Vorrichtung gemäß dem ersten Ausführungsbeispiel ist derart ausgebildet, dass sich das Messer 6 relativ zu der Schneidplatte 8 in die Hubrichtung 10 bewegen lässt, trotz der durch die ersten und zweiten Klemmen 12, 14 zwischen dem Oberteil 4, dem Messer 6, der Schneidplatte 8 und dem Unterteil 2 gebildeten Klemmverbindungen.

Dies ist unter anderem deshalb möglich, weil an den beiden Längsseiten des Messers 6 Längsausnehmungen 6.2 ausgebildet sind. Entsprechend wird eine Bewegung des Messers 6 parallel zu dem Pfeil 10 und in dem erforderlichen Bewegungsbereich durch die zweiten Klemmen 14 nicht behindert. Ein in die Hubrichtung 10 bestimmter Messerhub ist dabei durch die Länge der Längsausnehmungen 6.2 begrenzt. Die Zuordnung der Schneidlöcher 6.1 des Messers 6 einerseits zu den Schneidlöchern 8.1 der Schneidplatte 8, den Bodenöffnungen 4.1.1 und den Probenbehälteröffnungen andererseits ist dabei bevorzugt so gewählt, dass in einer ersten Hubendstellung des Messers 6 die Schneidlöcher 6.1, 8.1 derart übereinander liegen, dass die Pflanzen während der Pflanzenentwicklung ungehindert von dem Anzuchtbehälter 4.1 in den Probenbehälter 2.1 wachsen können, und dass die Wurzeln der Pflanzen in der zweiten Hubendstellung durchtrennt sind. Das Durchtrennen der Wurzeln erfolgt hierbei kontaminationsfrei, sofern der Messerhub kleiner gewählt ist als ein in die Hubrichtung 10 des Messers 6 bestimmter Abstand benachbarter Schneidlöcher 6.1, 8.1 und/oder Probenbehälter 2.1.

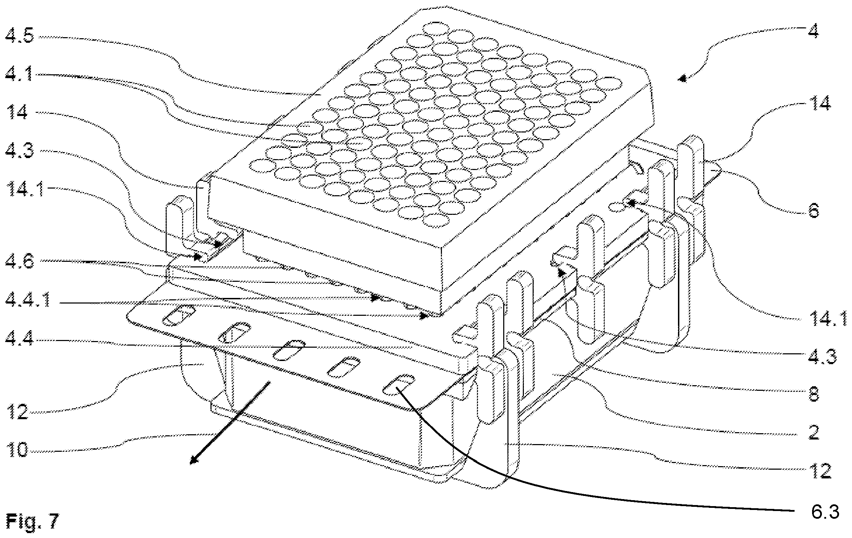

In Fig. 7 ist ein zweites Ausführungsbeispiel der erfindungsgemäßen Probennahme-Vorrichtung in einer Zusammenbaulage gezeigt. Im Unterschied zu dem ersten Ausführungsbeispiel weist das zweite Ausführungsbeispiel ein zweiteiliges Oberteil 4 auf. Das Oberteil 4 umfasst hier eine Basisplatte 4.4 und einen die Anzuchtbehälter 4.1 tragenden Aufsatz 4.5, wobei der Aufsatz 4.5 und die Basisplatte 4.4 in einer in Fig. 7 dargestellten Gebrauchslage miteinander lösbar verbunden sind.

Die Zweiteilung des Oberteils 4 dient dazu, eine Mehrfachbenutzung der Probennahme-Vorrichtung zu erleichtern oder den Aufsatz 4.5 mit den Anzuchtbehältern 4.1 als vorkonfektioniertes Teil zu verwenden.

Bei dem vorliegenden Ausführungsbeispiel ist die Bodenöffnung jedes Anzuchtbehälters 4.1 des Aufsatzes 4.5 außenseitig mit einem rohrartigen Kragen 4.6 umgeben, wobei der Kragen 4.6 in der in Fig. 7 dargestellten Zusammenbauposition der Probennahme-Vorrichtung im Wesentlichen bis an das dem Aufsatz 4.5 abgewandte Ende der Basisplatte 4.4 reicht.

Um den Aufsatz 4.5 an der Basisplatte 4.4 zu befestigen, wird der Aufsatz 4.5 mit den rohrartigen Kragen 4.6 in an der Basisplatte 4.4 ausgebildete Durchgangslöcher 4.4.1 gesteckt. Die Kragen 4.6 und die dazu korrespondierenden Durchgangslöcher 4.4.1 sorgen gleichzeitig für eine Positionierung des Aufsatzes 4.5 zu der Bodenplatte 4.4.

Der Aufsatz 4.5 und die Basisplatte 4.4 sind bevorzugt aus Kunststoff hergestellt.

Die auf diese Weise gebildete Baueinheit aus Bodenplatte 4.4 und dem die Anzuchtbehälter 4.1 aufweisenden Aufsatz 4.5 ist in der in Fig. 7 dargestellten Gebrauchslage, wie im Rahmen des ersten Ausführungsbeispiels bereits erläutert, mittels zweiter Klemmen 14 mit den übrigen Bauteilen, nämlich dem die Probenbehälter 2.1 aufweisenden Unterteil 2, dem Messer 6 und der Schneidplatte 8 lösbar verbunden. Analog zu dem ersten Ausführungsbeispiel sind das Unterteil 2 und die Schneidplatte 8 auch hier vorab mittels erster Klemmen 12 miteinander verbunden.

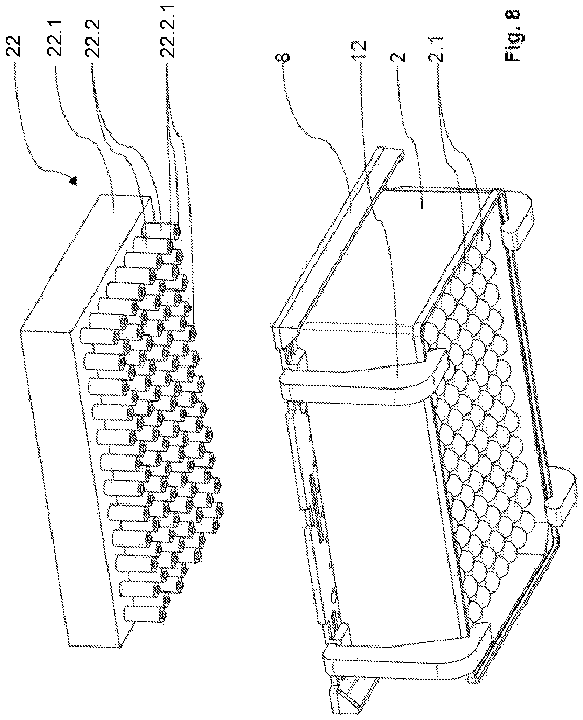

In den Fig. 8 bis 11 ist ein drittes Ausführungsbeispiel der Probennahme-Vorrichtung erläutert.

Fig. 8 zeigt das dritte Ausführungsbeispiel in einer perspektivischen Explosionsdarstellung in teilweiser Ansicht. Dargestellt ist die Baugruppe aus dem Unterteil 2 mit den Probenbehältern 2.1, den ersten Klemmen 12 und der Schneidplatte 8. Das Unterteil 2 mit den Probenbehältern 2.1 und die ersten Klemmen 12 wie auch die nicht dargestellten Bauteile der Probennahme-Vorrichtung, wie beispielsweise das Oberteil 4 und das Messer 6, können analog zu dem ersten oder dem zweiten Ausführungsbeispiel ausgebildet sein.

Der Probennahme-Vorrichtung ist vorliegend zusätzlich ein weiteres Bauteil zugeordnet, nämlich einen Stanzstempel 22 einer Stanzeinrichtung 20, die hier nicht dargestellt ist und auf die nachfolgend noch im Detail eingegangen wird. Aus einer Basisplatte 22.1 des Stanzstempels 22 erheben sich Stifte 22.2, die jeweils einen Positionierkopf 22.2.1 aufweisen. Die Anzahl der Stifte 22.2 stimmt mit der Anzahl der Probenbehälter 2.1 und damit mit der Anzahl der hier nicht dargestellten Anzuchtbehälter überein. Der Stanzstempel 22 ist vorzugsweise aus einem metallischen Werkstoff oder aus Kunststoff hergestellt.

Der Stanzstempel 22 dient dazu, eine Kreuzkontamination der Wurzelproben beim Abheben der Schneidplatte 8 sicher zu vermeiden. Hierzu werden nach dem Schneiden der Wurzeln und dem Entfernen des nicht dargestellten einteiligen oder zweiteiligen Oberteils 4 mittels des Stanzstempels 22 rund um die einzelnen Schneidlöcher 8.1 kreisringförmige Abschnitte 8.5 aus der Schneidplatte 8 herausgestanzt und in das Innere der jeweils zugeordneten Probenbehälter 2.1 überführt. Dort können Sie auch während der sich anschließenden labortechnischen Untersuchung verbleiben.

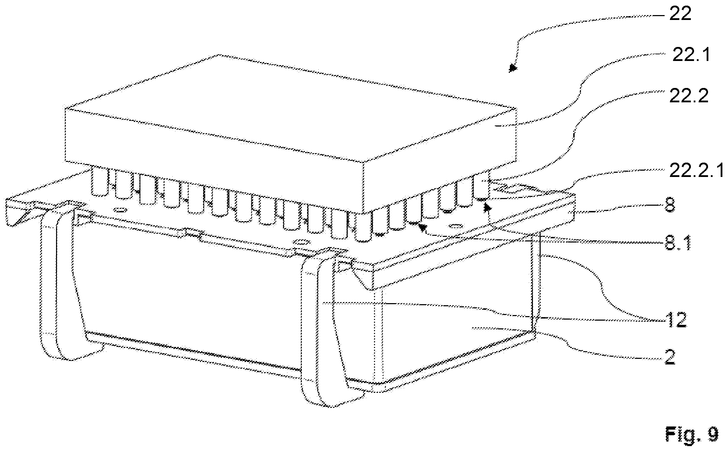

Wie in Fig. 9 dargestellt wird hierfür der Stanzstempel 22 mit der Schneidplatte 8 in Eingriff gebracht. Um eine sichere Ausrichtung des Stanzstempels 22 mit dessen Stiften 22.2 zu der Schneidplatte 8 und den Schneidlöchern 8.1 zu gewährleisten und zu erleichtern, gelangen bei der Annäherung des Stanzstempels 22 an die Schneidplatte 8 zunächst die an den Stiften 22.2 ausgebildeten Positionierköpfe 22.2.1 in Eingriff mit den Schneidlöchern 8.1 der Schneidplatte 8. Bei der weiteren Bewegung des Stanzstempels 22 in Richtung Schneidplatte 8 gelangen die Stifte 22.2 des Stanzstempels 22 in Anlage mit Rändern der Schneidlöcher 8.1 der Schneidplatte 8.

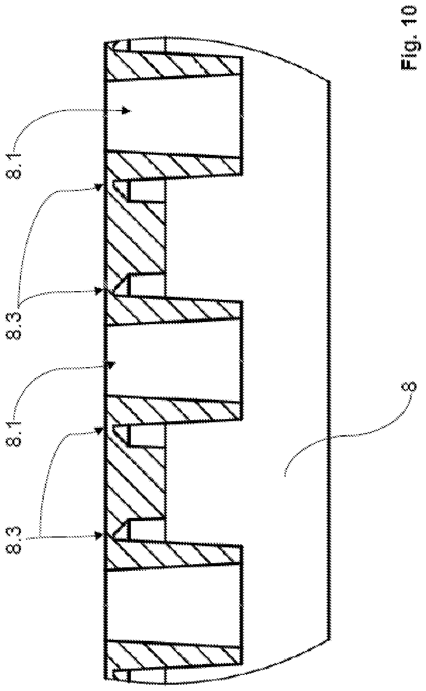

In Fig. 10 ist ein Detail der Probennahme-Vorrichtung gemäß dem dritten Ausführungsbeispiel im Bereich der Schneidplatte 8 gezeigt ist. Dargestellt sind die Ränder 8.3, die die Schneidlöcher 8.1 der Schneidplatte 8 umgeben. Die Dicke der Schneidplatte 8 ist an den Rändern 8.3 geschwächt, so dass der hier nicht dargestellte Stanzstempel 22 bei der weiteren Bewegung in Richtung der Schneidplatte 8 die so gebildeten Sollbruchstellen der Schneidplatte 8 bricht. Der Stanzstempel 22 wird bei der Bewegung in Richtung der Schneidplatte 8 in der Bildebene von Fig. 10 manuell oder motorisch von oben auf die Schneidplatte 8 abgesenkt.

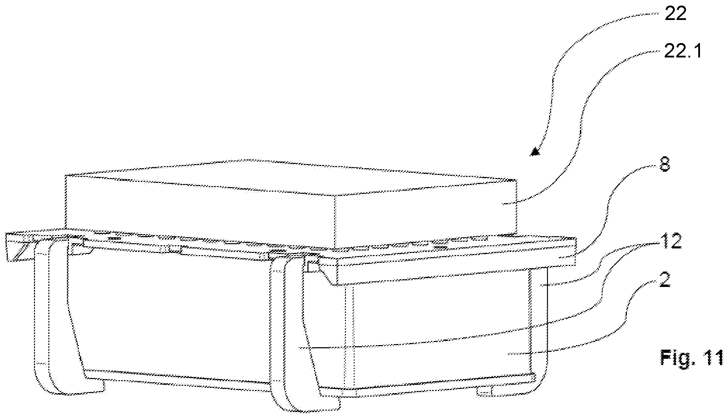

Die einzelnen Stifte 22.2 des Stanzstempels 22 weisen eine entsprechende Dimensionierung auf, um die Ränder 8.3 der Schneidplatte 8 mit den darin ausgebildeten Schneidlöchern 8.1 bei der beschriebenen Bewegung des Stanzstempels 22 in Richtung der Schneidplatte 8 sicher in das Innere des jeweils korrespondierenden Probenbehälters 2.1 zu überführen und in dem Probenbehälter 2.1 zu halten, um ein ungewünschtes Herausziehen von Wurzelproben aus den Probenbehältern 2.1 bei einer Entfernung der Schneidplatte 8 von dem Unterteil 2 und damit von den Probenbehältern 2.1 wirksam zu verhindern. Das Unterteil 2 und die Probenbehälter 2.1 sind in Fig. 10 ebenfalls nicht dargestellt. Fig. 11 zeigt den Stanzstempel 22 in der Endlage (Betätigungsposition), in der der Stanzstempel 22 mit dessen Basisplatte 22.1 an der Schneidplatte 8 anliegt beziehungsweise zu diesem benachbart vorgesehen ist. Der Übersichtlichkeit wegen ist die Basisplatte 2.1 in der Fig. 11 nicht vollständig auf die Schneidplatte 8 abgesenkt dargestellt.

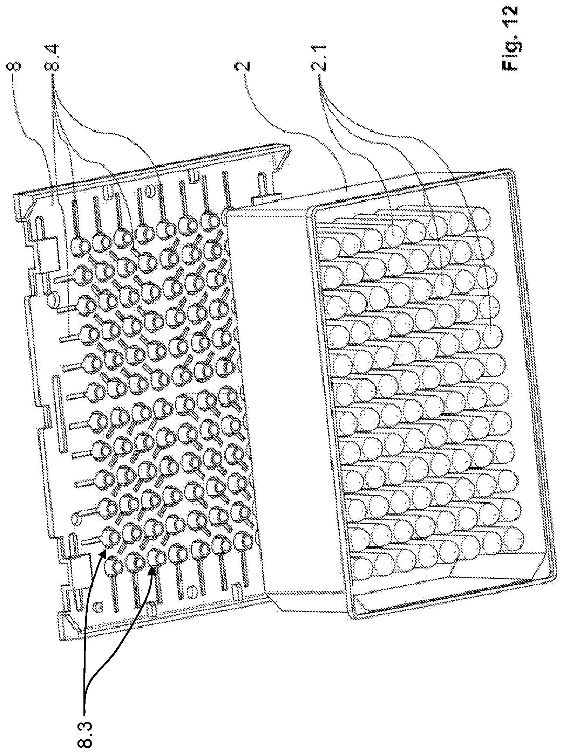

In Fig. 12 ist ferner ein viertes Ausführungsbeispiel dargestellt. Gezeigt ist das Unterteil 2 mit den Probenbehältern 2.1 und die Schneidplatte 8 in einer perspektivischen Unteransicht. Das Unterteil 2 mit den Probenbehältern 2.1 wie auch die nicht dargestellten Bauteile der Probennahme-Vorrichtung, wie beispielsweise das Oberteil 4 und das Messer 6, können analog zu dem ersten oder dem zweiten oder dem dritten Ausführungsbeispiel ausgebildet sein.

Im Unterschied zu den bereits erläuterten Ausführungsbeispielen weist die Probennahme-Vorrichtung des vierten Ausführungsbeispiels eine modifizierte Schneidplatte 8 auf. Wie aus Fig. 12 ersichtlich ist, sind in der Unterseite der Schneidplatte 8 die Ränder 8.3 und als Nuten ausgebildete Entwässerungsöffnungen 8.4 ausgebildet. Jede der Entwässerungsöffnungen 8.4 ist dabei in der nicht dargestellten Zusammenbaulage von Unterteil 2 und Schneidplatte 8 genau einem Probenbehälter 2.1 zugeordnet, um so eine ungewünschte Kreuzkontamination wirksam zu verhindern.

Wie bereits anhand des ersten Ausführungsbeispiels erläutert, werden in den in Fig. 12 nicht dargestellten Anzuchtbehältern 4.1 Pflanzen gezogen. Hierfür sind die Anzuchtbehälter 4.1 mit Nährstoffen für das Pflanzenwachstum befüllt. Um die erforderliche Menge an Wasser für das Wachstum zu speichern, kann in den Anzuchtbehältern 4.1 ein Granulat oder dergleichen eingebracht sein. Die Anzuchtbehälter 4.1 werden bevorzugt von oben gewässert, um so das Granulat mit Wasser zu tränken, welches dann von dem Granulat an die Pflanzen abgegeben wird. Bei dem Wässern kann es vorkommen, dass ein Überschuss an Wasser den einzelnen Anzuchtbehältern 4.1 zugeführt wird. Dieses Wasser kann nicht von dem Granulat aufgenommen werden; es läuft über die Bodenöffnungen der betroffenen Anzuchtbehälter in die diesen zugeordneten Probenbehälter 2.1.

Für die Anzucht ist es gewünscht beziehungsweise unschädlich, dass sich in den Probenbehältern 2.1 Wasser befindet. Jedoch ist es ungewünscht, dass die Anzuchtbehälter 4.1 von Wasser geflutet sind. Deshalb ist in dem vierten Ausführungsbeispiel für jeden Probenbehälter 2.1 die als Nut ausgebildete Entwässerungsöffnung 8.4 vorgesehen. Die Nuten 8.4 sind dabei derart an der Unterseite der Schneidplatte 8 angeordnet, dass Wasser, welches von dem einzelnen Probenbehälter 2.1 nicht aufgenommen werden kann, nicht in ungewünschter Weise in den dazu korrespondierenden Anzuchtbehälter 4.1 aufsteigt, sondern über die nicht dargestellte Probenbehälteröffnung in die jeweilige Nut 8.4 gelangt und über die Unterseite der Schneidplatte 8 abfließen kann, ohne in ungewünschter Weise in einen der anderen Probenbehälter 2.1 einzufließen. Auf diese Weise ist Staunässe wirksam vermieden und der notwendige Gasaustausch ist gewährleistet.

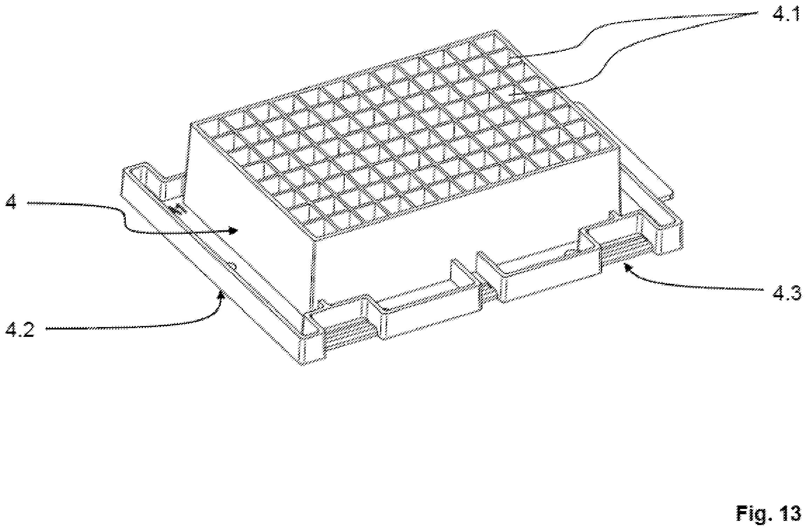

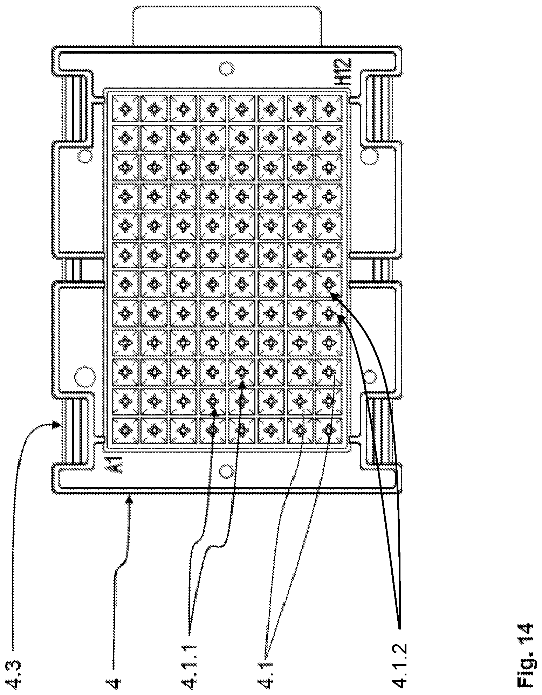

Anhand der Fig. 13 und 14 ist ein fünftes Ausführungsbeispiel der Probennahme-Vorrichtung erläutert. Fig. 13 zeigt das fünfte Ausführungsbeispiel in einer perspektivischen Explosionsdarstellung in einer teilweisen Ansicht. Dargestellt ist das Oberteil 4 mit den Anzuchtbehältern 4.1, welche matrixförmig in einer 12x8-Anordnung vorgesehen sind. Die Anzuchtbehälter 4.1 sind nach dem fünften Ausführungsbeispiel anders als zuvor im Querschnitt rechteckförmig realisiert. Es ist durch den rechteckförmigen Querschnitt der Anzuchtbehälter 4.1 eine sehr gute Raumausnutzung gegeben beziehungsweise bei einer unveränderten Größe des Oberteils 4 kann das Volumen der Anzuchtbehälter 4.1 vergrößert werden.

Die an dem Oberteil 4 für jeden Anzuchtbehälter 4.1 vorgesehenen Bodenöffnungen 4.1.1 sind in Umfangsrichtung von jeweils eine Mehrzahl von Dornen 4.1.2 umgeben, die bezogen auf die Zusammenbauposition von dem Unterteil 2 der Probennahme-Vorrichtung wegweisen. Die Dorne 4.1.2 sind so beabstandet und angeordnet, dass einer Verstopfung beziehungsweise einem Verschluss der Bodenöffnung 4.1.1 durch die Nährstoffe entgegengewirkt ist und zugleich sichergestellt werden kann, dass die Wurzeln sich in der Anzuchtphase durch die Bodenöffnungen 4.1.1 in das Unterteil entwickeln können.

Selbstverständlich kann auch bei der rechteckförmigen Ausgestaltung der Anzuchtbehälter 4.1 nach dem fünften Ausführungsbeispiel das Oberteil 4 zweiteilig ausgebildet sein. Es weist dann das Oberteil 4 analog zur Realisierung der erfindungsgemäßen Probennahme-Vorrichtung gemäß Fig. 7 die Basisplatte 4.4 sowie den Aufsatz 4.5 mit den im Querschnitt rechteckigen Anzuchtbehältern 4.1 auf.

Für die Durchführung des erfindungsgemäßen Verfahrens können auch andere als die dargestellte Probennahme-Vorrichtung verwendet werden.

Um die einzelnen Bauteile der Probennahme-Vorrichtung, beispielsweise Unterteil 2, Oberteil 4, Messer 6 und Schneidplatte 8, mit weniger Kontrollaufwand zueinander sicher ausrichten zu können, können die Bauteile auch bei nicht zweigeteiltem Oberteil 4 zumindest teilweise zueinander korrespondierende Positioniermittel aufweisen.

Die Positioniermittel können ferner als Codierung ausgebildet sein, durch die eine fehlerhafte Montage der Bauteile der Probennahme-Vorrichtung mit einfachen Mitteln wirksam verhindert ist.

Bei den genannten Ausführungsbeispielen sind die Probenbehälter 2.1 des Unterteils 2 und die Anzuchtbehälter 4.1 des Oberteils 4 jeweils integraler Bestandteil des Unterteils 2 oder Oberteils 4 beziehungsweise des Aufsatzes 4.5. Dies ist jedoch nicht zwingend erforderlich. Beispielsweise kann es auch vorgesehen sein, dass die Probenbehälter 2.1 und/oder die Anzuchtbehälter 4.1 zumindest teilweise als separates Bauteil ausgebildet sind.

Die Probennahme-Vorrichtung kann sowohl für den einmaligen Gebrauch wie auch für den mehrmaligen Gebrauch ausgelegt sein. Während die erste Ausführungsform eher als Einweg-Probenahme-Vorrichtung geeignet ausgebildet ist, eignet sich die zweite Ausführungsform besser für die mehrfache Verwendung.

Im Unterschied zu den Ausführungsbeispielen wäre es grundsätzlich denkbar, dass ein Anzuchtbehälter 4.1 mit dessen Bodenöffnung 4.1.1 nicht zwingend genau zu einem Probenbehälter 2.1 und dessen Probenbehälteröffnung korrespondiert. Möglich wäre auch, dass ein Anzuchtbehälter 4.1 mit dessen Bodenöffnung einer Mehrzahl von Probenbehältern 2.1 und deren Probenbehälteröffnungen zugeordnet ist. Gleiches Pflanzenmaterial kann so unterschiedlichen Untersuchungen zugeführt werden.

Das Messer 6 muss nicht zwingend als eine Lochplatte ausgebildet sein. Denkbar ist auch, dass beispielsweise lediglich die Schneidplatte 8 als eine Lochplatte ausgebildet ist und das Messer 6 von dem Fachmann je nach dem vorliegenden Einzelfall nach Art, Material, Form, Dimensionierung und Anordnung anders geeignet ausgewählt ist.

Beispielsweise wären, neben anderen geeigneten Materialien, Messer 6 aus gehärtetem Werkzeugstahl, aus legiertem Werkzeugstahl, aus Hartmetall, aus Kunststoff oder auch aus Schneidkeramik möglich. Gleiches gilt für das Material der Schneidplatte 8.

In den beiden Ausführungsbeispielen wirken die zweiten Klemmen 14 in der Zusammenbauposition der Probennahme-Vorrichtung unter anderem mit an dem Unterteil 2 ausgebildeten Rastaufnahmen 2.3 zusammen. Da das Unterteil 2 und die Schneidplatte 8 jedoch mit ersten Klemmen 12 miteinander lösbar verbunden sind, wäre es auch denkbar, dass die zweiten Klemmen 14 mit an der Schneidplatte 8 ausgebildeten Rastaufnahmen 8.2 zusammenwirken.

Das erfindungsgemäße Anzucht- und Probennahme-Verfahren sieht nunmehr beispielsweise vor, dass eine Pflanze in dem Anzuchtbehälter 4.1 herangezogen wird. Es ist hierzu so, dass das Substrat beziehungsweise die Nährstoffe in die Anzuchtbehälter 4.1 des Oberteils 4 der Probennahme-Vorrichtung eingebracht werden beziehungsweise das Oberteil 4 oder die gesamte Probennahme-Vorrichtung mit dem bereits hierin befindlichen Substrat beziehungsweise Nährstoffe vorkonfektioniert bereitgestellt und das Saatgut in die Anzuchtbehälter 4.1 eingebracht wird.

Es werden dann nach einer Anzuchtphase, in der sich die Pflanze entwickeln und die Wurzeln der Pflanze durch die Bodenöffnung 4.1.1 des Anzuchtbehälters 4.1, die Schneidlöcher 6.1 des Messers 6, die Schneidlöcher 8.1 der Schneidplatte 8 und die Probenbehälteröffnungen in die korrespondierenden Probenbehälter 2.1 wachsen, die in dem Unterteil 2 vorgesehenen Wurzelteile mit dem Messer 6 von den Pflanzen abgetrennt.

Während einer nachfolgenden Analysephase wird das Unterteil 2 der Probennahme-Vorrichtung mit den darin befindlichen Wurzelteilen einer Analyseeinrichtung zugeführt. Das Oberteil der Probennahme-Vorrichtung mit den vitalen, das heißt intakten und voll funktionsfähigen (Rest-) Pflanzen kann bis zum Vorliegen der Analyseergebnisse und/oder bis zur Selektion weiter kultiviert werden. In der Analyseeinrichtung werden an den Wurzelteilen phänotypische Beschreibungen und/oder molekularbiologische Untersuchungen durchgeführt. Nach der Durchführung der Analyse wird ermittelt, welche Pflanzen hinsichtlich bestimmter Spezifikationsmerkmale besonders günstige, gewünschte Eigenschaften aufweisen. Beispielsweise kann dies eine Kältetoleranz, eine Schädlingsresistenz oder Ähnliches sein.

Nachdem die entsprechenden Pflanzen identifiziert sind, wird das Oberteil 4 der Probennahme-Vorrichtung einer Selektionseinrichtung zugeführt. Das Oberteil 4 der Probennahme-Vorrichtung beziehungsweise der Aufsatz 4.5 des Oberteils 4 in einer Aufnahme 31 eines Signalgebers 30 der Selektionseinrichtung an einer definierten Stelle positioniert. Ein Anzuchtbehälter 4.1 der Probennahme-Vorrichtung wird dann mittels des Signalgebers 30 optisch gekennzeichnet. Der Signalgeber 30 sieht hierzu ein Leuchtdioden-Array 32 vor, welches eine Vielzahl von Leuchtdioden aufweist, die korrespondierend zu den Anzuchtbehältern 4.1 angeordnet sind. In dem gekennzeichneten Anzuchtbehälter 4.1 befindet sich insofern die die besonders günstigen Eigenschaften aufweisende Pflanze. Wenigstens ein Teil der in dem Anzuchtbehälter 4.1 vorgesehenen Pflanze wird dann entnommen zur weiteren Verarbeitung.

Im Rahmen des erfindungsgemäßen Anzucht- und Probennahme-Verfahrens kann zur automatischen oder manuellen maschinellen Betätigung des Messers 6 eine Trenneinrichtung 40 vorgesehen sein, welche in einer perspektivischen Einzeldarstellung in Fig. 15 gezeigt ist. Die Trenneinrichtung 40 sieht einen Betätigungshebel 41 sowie einen über einen Exzenter 42 mit dem Betätigungshebel 41 verbundenen, linear verstellbaren Hubabschnitt 43 vor. Der Hubabschnitt 43 ist in einer in Fig. 15 dargestellten Grundposition der Trenneinrichtung 40 in einer definierten, vorbestimmten Lage relativ zu einer Anlagefläche 44 der Trenneinrichtung 40 vorgesehen.

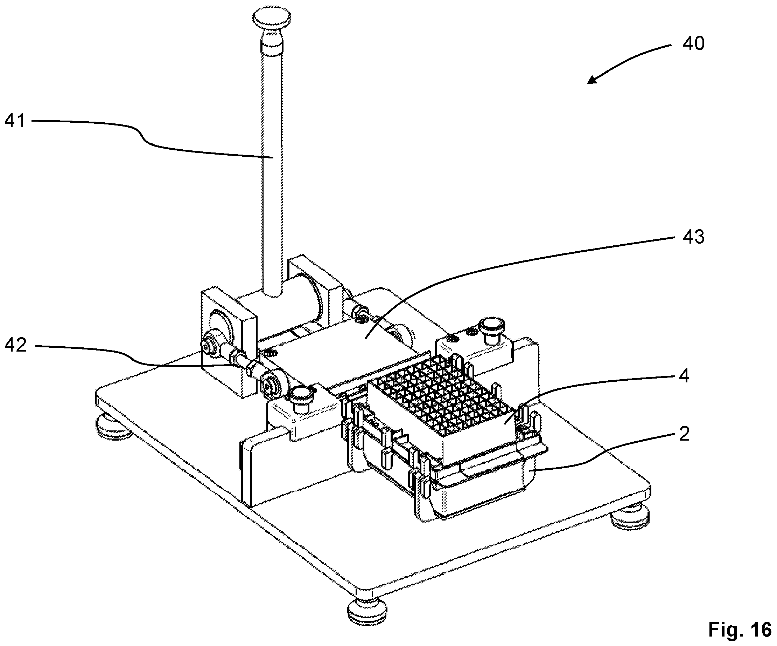

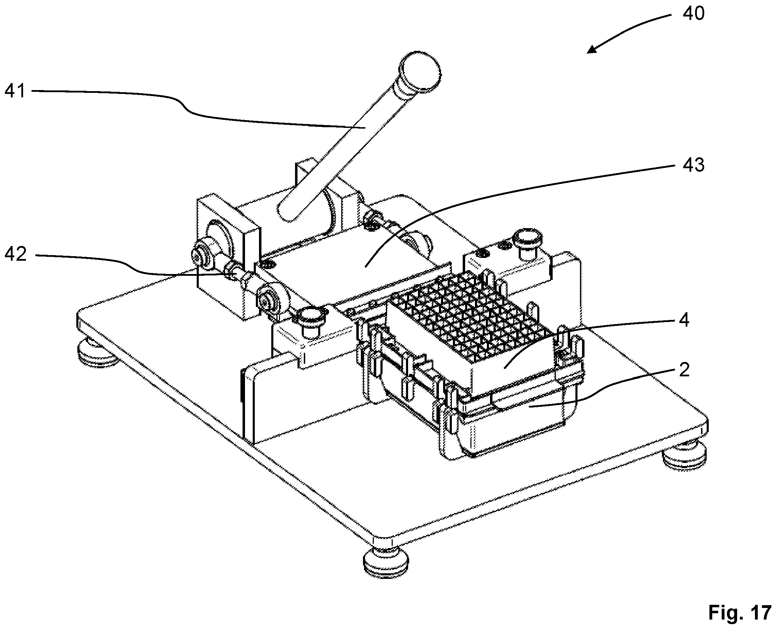

Die Fig. 16 und 17 zeigen die Trenneinrichtung 40 mit der darin eingesetzten Probennahme-Vorrichtung. Es ist insbesondere so, dass die Probennahme-Vorrichtung gegen die Anlagefläche 44 positioniert ist und das Messer 6 der Probennahme-Vorrichtung mit dem Hubabschnitt 43 der Trenneinrichtung 40 verbunden ist. Zu diesem Zweck sind an dem Hubabschnitt 43 Aufnahmestifte 45 vorgesehen, die in die an dem Messer 6 vorgesehenen Aufnahmen 6.3 eingreifen. Sofern die Trenneinrichtung 40 nun durch Betätigung des Betätigungshebels 41 aus der Grundposition in eine Betätigungsposition verbracht wird, wird das Messer 6 der Probennahme-Vorrichtung in die Hubrichtung 10 längsverschoben und die Wurzeln der Pflanzen werden durchtrennt.

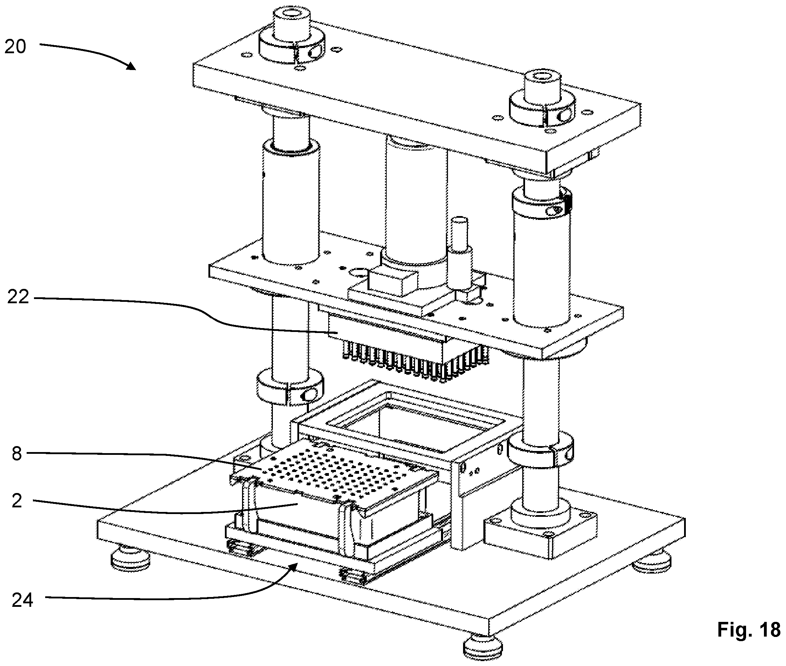

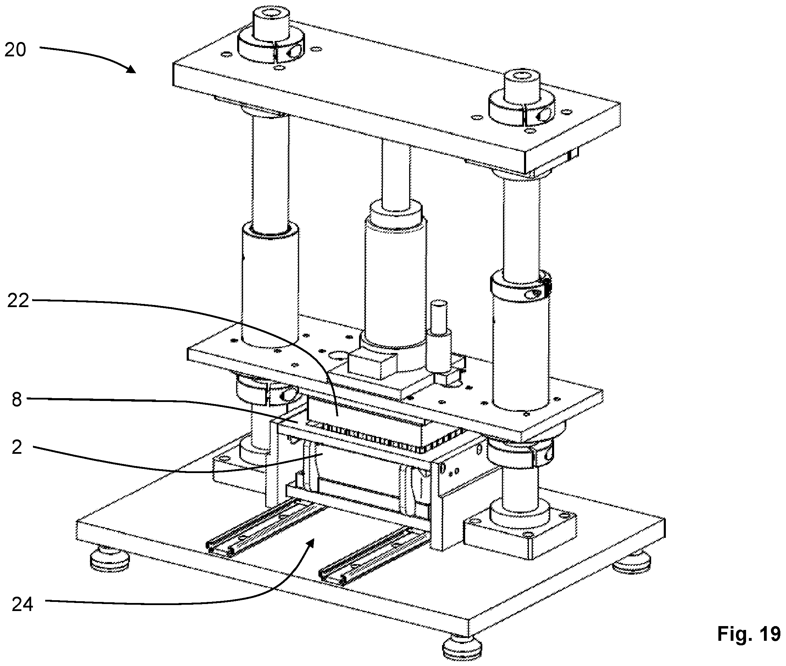

Nach dem Durchtrennen der Wurzeln kann in Vorbereitung der Analyse der Pflanzen das Unterteil 2 der Probennahme-Vorrichtung zusammen mit der daran festgelegten Schneidplatte 8 der in den Fig. 18 und 19 dargestellten Stanzeinrichtung 20 zugeführt. Die Stanzeinrichtung 20 umfasst den linear verschiebbaren Stanzstempel 22 mit der Basisplatte 22.1 sowie den Stiften 22.2 und den an den Stiften 22.2 vorgesehenen Positionierköpfen 22.2.1. Darüber hinaus umfasst die Stanzeinrichtung 20 eine Aufnahme 24, in die das Unterteil 2 der Probennahme-Vorrichtung zusammen mit der Schneidplatte 8 eingesetzt und längsverschoben werden kann. Der Stanzstempel 22 der Stanzeinrichtung 20 wird beispielsweise motorisch, insbesondere elektromotorisch oder-wie dargestellt ― pneumatisch beziehungsweise hydraulisch linear betätigt und aus einer oberen Grundposition gemäß Fig. 18 nach dem Verbringen der Probennahme-Vorrichtung unter den Stanzstempel 22 in eine Betätigungsposition verbracht.

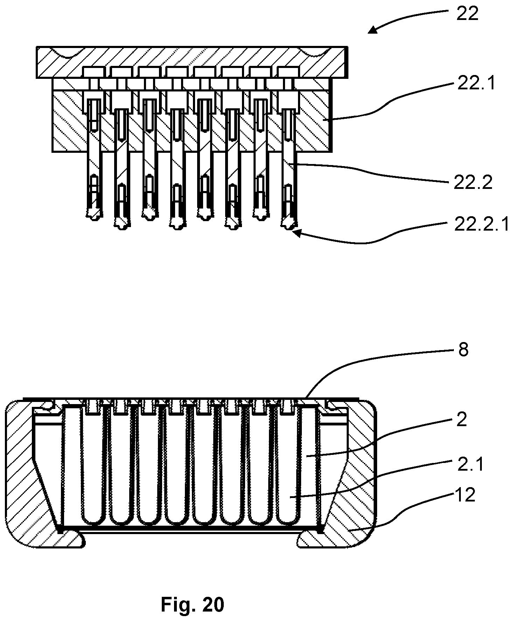

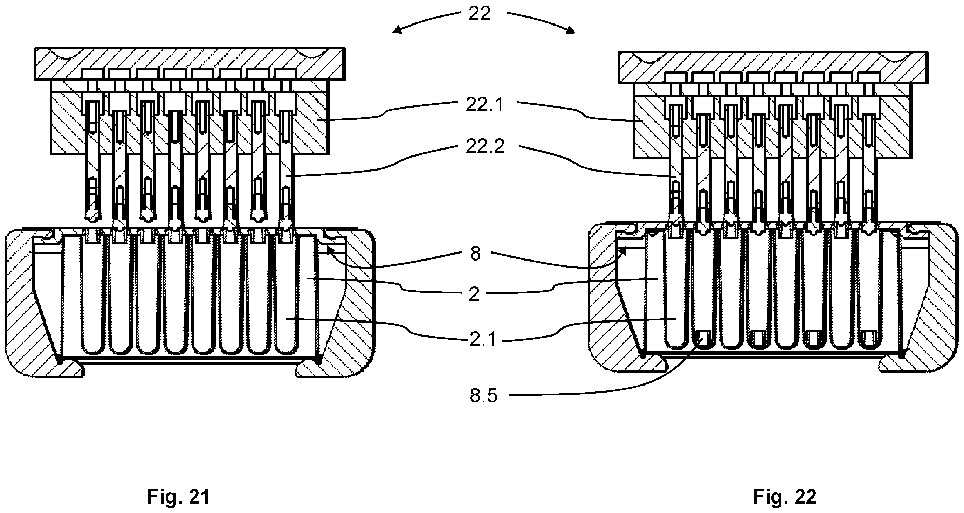

Die einzelnen Schritte beim Stanzen sind sequenziell in den Fig. 20 bis 24 dargestellt. Gemäß Fig. 20 ist der Stanzstempel 22 in der oberen Grundposition vorgesehen und das Unterteil 2, welches über die erste Klemmen 12 mit der Schneidplatte 8 verbunden ist, unterhalb des Stanzstempels 22 positioniert. Im Weiteren wird der Stanzstempel 22 abgesenkt, bis jedenfalls erste Positionierköpfe 22.2.1 der Stifte 22.2 in erste Schneidlöcher 8.1 der Schneidplatte 8 eingreifen (Fig. 21). Wird der Hub fortgesetzt, werden durch die ersten Stifte 22.2 erste ringförmige Abschnitte 8.5 aus der Schneidplatte 8 herausgestanzt und in die zugeordneten Probenbehälter 2.1 überführt, vergleiche Fig. 22. Hierbei gelangen die weiteren Positionierköpfe 22.2.1 der Stifte 22.2 in Eingriff mit den weiteren Schneidlöchern 8.1 der Schneidplatte 8.

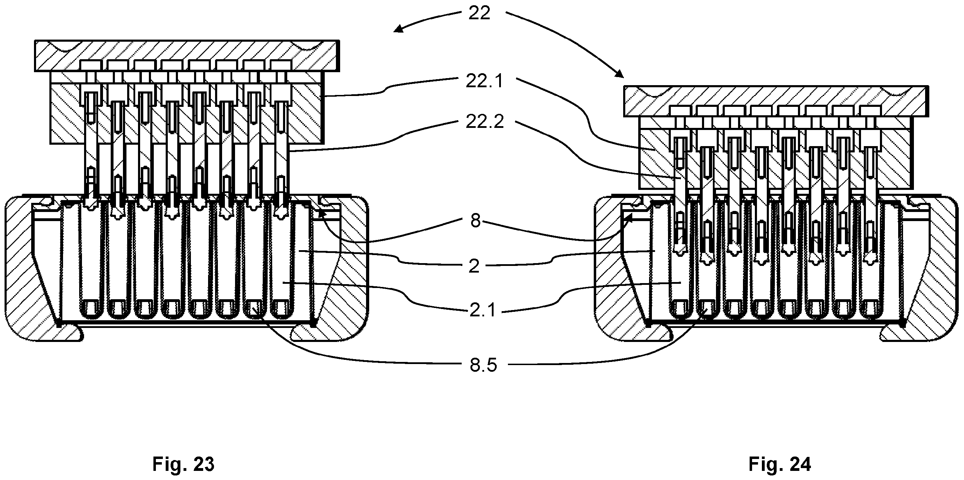

Bei einer Fortsetzung der Stanzbewegung werden, wie in Fig. 23 dargestellt, die verbleibenden ringförmigen Abschnitte 8.5 aus der Schneidplatte 8 herausgestanzt und in die Probenbehälter 2.1 überführt. Sobald der Stanzstempel 22 seine untere Grundstellung gemäß Fig. 24 eingenommen hat, ist der Stanzvorgang beendet. Die Wurzelteile befinden sich zusammen mit den Abschnitten 8.5 sicher verwahrt in den Probenbehältern 2.1 und die Schneidplatte 8 kann nach dem Entfernen der ersten Klemmen 12 von dem Unterteil 2 der Probennahme-Vorrichtung entfernt werden.

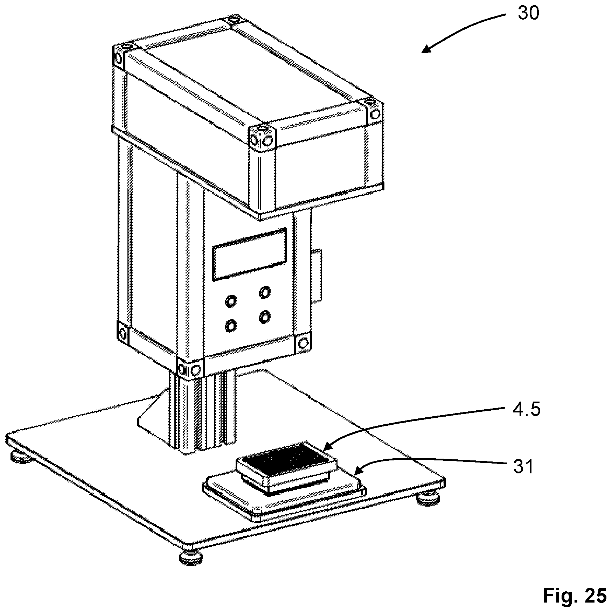

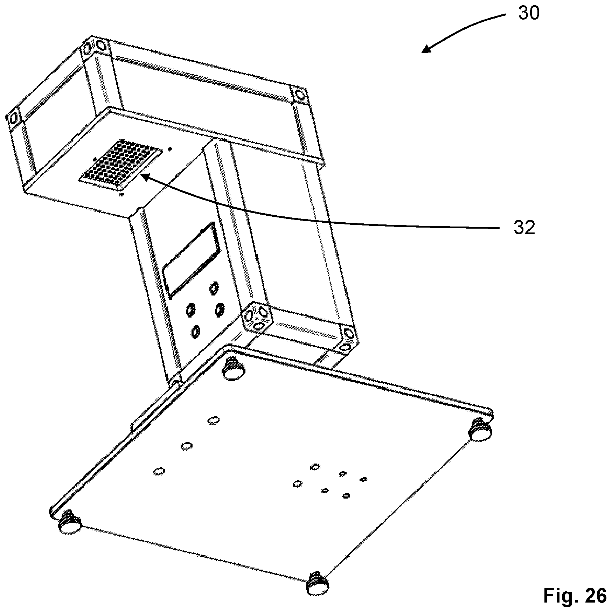

Im Rahmen einer lasergestützten Aussaat des Saatguts der Pflanzen und/oder einer lasergestützten Selektion der Pflanzen nach der Analyse kommt ein Signalgeber 30 zum Einsatz, welcher in einer perspektivischen ersten Darstellung in Fig. 25 dargestellt ist. Der Signalgeber 30 umfasst eine Aufnahme 31 für das Oberteil 4 beziehungsweise den Aufsatz 4.5 des Oberteils 4 der Probennahme-Vorrichtung. Zusätzlich umfasst der Signalgeber 30 eine Vielzahl von Leuchtdioden, welche in einem in der Fig. 26 dargestellten Leuchtdioden-Array 32 der Aufnahme 31 gegenüberliegend angeordnet sind. Durch die Leuchtdioden können einzelne Anzuchtbehälter 4.1 des Oberteils 4 bei der Aussaat und/oder der Selektion optisch gekennzeichnet werden. Die optische Kennzeichnung der Anzuchtbehälter 4.1 stellt eine Führung für das Laborpersonal dar und wirkt einer fehlerhaften Aussaat oder Entnahme entgegen.

Beispielsweise läuft das erfindungsgemäße Anzucht- und Probennahme-Verfahren so ab, dass zunächst mithilfe einer den Signalgeber 30 aufweisenden Aussaateinrichtung Saatgut für die Pflanzen in die verschiedenen Anzuchtbehälter 4.1 eingebracht wird. Hierbei wird in einer Datenbank gespeichert, welches Saatgut in welchem Anzuchtbehälter 4.1. hinterlegt ist. Zudem wird eine Oberteilkennzeichnung des Oberteils 4 sowie korrespondierend hierzu eine Unterteilkennzeichnung des Unterteils 2 der Probennahme-Vorrichtung gespeichert.

In der Anzuchtphase werden dann die Pflanzen entwickelt. Die Anzucht erfolgt in einem Kulturraum, beispielsweise einem Gewächshaus. Bevorzugt kann vorgesehen sein, dass die Pflanzen während der Anzucht mit einem speziellen Licht einer Leuchtdioden-Beleuchtungsanordnung beleuchtet werden. Es hat sich hier gezeigt, dass durch eine entsprechende Beleuchtung die Entwicklung der Wurzeln begünstigt und das Wachstum des Sprosses der Pflanzen gehemmt werden kann.

Nachdem eine ausreichende Entwicklung der Pflanzen erfolgt ist, werden die Wurzeln mittels der Trenneinrichtung 40 durchtrennt und das Unterteil 2 zusammen mit der Schneidplatte 8 der Stanzeinrichtung 20 zugeführt zum Ausstanzen der Abschnitte 8.5 aus der Schneidplatte 8. Im Weiteren folgt nach der Entfernung der Schneidplatte 8 von dem Unterteil 2 die Analyse der in dem Unterteil 2 vorgesehenen Wurzelteile in einer Analyseeinrichtung. Beispielsweise wird eine RNA- und/oder eine DNA-Analyse an den Wurzelteilen durchgeführt. Die herausgestanzten Abschnitte 8.5 können während dieser Analyse in den Probenbehältern 2.1 des Unterteils 2 verbleiben.

Nach der Durchführung der Analyse werden Pflanzen identifiziert, welche einer bevorzugten Spezifikation in Bezug auf bestimmte Merkmale sehr gut entsprechen. Um mit den entsprechenden Pflanzen die weitere Entwicklung durchführen zu können, müssen nunmehr die besonders vorteilhaften Pflanzen selektiert werden. Es wird also das Oberteil 4 beziehungsweise der Aufsatz 4.5 des Oberteils 4 der Probennahme-Vorrichtung einer Selektionseinrichtung zugeführt und positioniert. Weiter werden die Oberteilkennzeichung erfasst und mittels des Signalgebers 30 der Anzuchtbehälter optisch gekennzeichnet, in dem sich die als besonders vorteilhaft identifizierte Pflanze befindet. Die Pflanze kann dann vollständig oder in Teilen entnommen werden. Insbesondere kann die Pflanze selbst umgetopft beziehungsweise verpflanzt werden. Hier ist im Rahmen des erfindungsgemäßen Anzucht- und Probennahme-Verfahrens typischerweise allein die Primärwurzel durchtrennt. Laterale Wurzeln können die Funktion der Primärwurzel übernehmen, sodass die Pflanze unmittelbar zur weiteren Züchtung verwendet werden kann.

Nach einer alternativen Ausführungsform des erfindungsgemäßen Verfahrens kann anstelle der optischen Kennzeichnung der Anzuchtbehälter 4.1 bei der Aussaat beziehungsweise Selektion oder zusätzlich zu der Kennzeichnung die Aussaat beziehungsweise die Entnahme der Pflanzensprosse automatisiert beziehungsweise teilautomatisiert erfolgen. Die Aussaateinrichtung und/oder die Selektionseinrichtung können zu diesem Zweck einen Greifer vorsehen, welcher bevorzugt automatisiert positioniert wird und dann das Saatgut in den ausgewählten Anzuchtbehälter 4.1 einbringt beziehungsweise jedenfalls Teile des Pflanzensprosses aus dem Anzuchtbehälter 4.1 entnimmt. Beispielsweise kann der Greifer in zwei Koordinaten über dem Oberteil 4 der Probennahme-Vorrichtung verfahren und positioniert werden.

Das erfindungsgemäße Anzucht- und Probennahme-Verfahren kann durchgängig oder in Teilen automatisiert vorgesehen werden. Beispielsweise können die verschiedenen Funktionseinrichtungen steuerungstechnisch beziehungsweise datentechnisch verknüpft sein. Insbesondere kann eine kontinuierliche Kontrolle und Identifikation während des gesamten Verfahrens gewährleistet werden, indem die Saatgutkennzeichnung, die Oberteilkennzeichnung der Probennahme-Vorrichtung sowie die Unterteilkennzeichnung der Probennahme-Vorrichtung in den verschiedenen Verfahrensschritten erfasst und/oder gespeichert und/oder kontrolliert werden und insbesondere abgespeichert wird, welches Saatgut in welchem Anzuchtbehälter 4.1 der Probennahme-Vorrichtung eingebracht wird, welche Wurzeln in welchem Probenbehälter 2.1 der Probennahme-Vorrichtung gewachsen sind, welche Spezifikation beziehungsweise welche Eigenschaften die Pflanzen aufweisen und sprosse aufgrund ihrer besonders vorteilhaften Eigenschaften selektiert und weiterverwendet werden. Beispielsweise können die Aussaateinrichtung, die Trenneinrichtung 40, die Stanzeinrichtung 20 und/oder die Selektionseinrichtung Erfassungsmittel für die Saatgutkennzeichnung, die Oberteilkennzeichnung und/oder die Unterteilkennzeichnung aufweisen. Eine durchgängige Erfassung und Kontrolle der Pflanzen, Pflanzenteile beziehungsweise Probennahme-Vorrichtung ist damit während des gesamten erfindungsgemäßen Anzucht- und Probennahme-Verfahrens durchgängig gewährleistet.

### Bezugszeichenliste

- 2: Unterteil
- 2.1: Probenbehälter
- 2.2: Rastkragen des Unterteils
- 2.3: Rastaufnahmen des Unterteils
- 4: Oberteil
- 4.1: Anzuchtbehälter
- 4.1.1: Bodenöffnung des Anzuchtbehälters
- 4.1.2: Dorne
- 4.2: Boden des Oberteils, als Messerführung für das Messer ausgebildet
- 4.3: Rastaufnahmen des Oberteils
- 4.4: Basisplatte des Oberteils
- 4.4.1: Durchgangslöcher der Basisplatte
- 4.5: Aufsatz des Oberteils
- 4.6: Kragen, der die Bodenöffnung des Anzuchtbehälters umgibt
- 6: Messer, als Messerlochplatte ausgebildet
- 6.1: Schneidlöcher des Messers
- 6.2: Längsausnehmungen des Messers
- 6.3: Aufnahmen
- 8: Schneidplatte, als Loch-Schneidplatte ausgebildet
- 8.1: Schneidlöcher der Schneidplatte
- 8.2: Rastaufnahmen der Schneidplatte
- 8.3: Ränder der Schneidplatte, die die Schneidlöcher umgeben
- 8.4: Entwässerungsöffnungen der Schneidplatte
- 8.5: herausgestanzte Abschnitte der Schneidplatte
- 10: Hubrichtung des Messers
- 12: erste Klemmen
- 12.1: Rastvorsprünge der ersten Klemmen
- 14: zweite Klemmen
- 14.1: Rastvorsprünge der zweiten Klemmen
- 20: Stanzeinrichtung
- 22: Stanzstempel
- 22.1: Basisplatte des Stanzstempels
- 22.2: Stifte des Stanzstempels
- 22.2.1: Positionierköpfe der Stifte
- 24: Aufnahme
- 30: Signalgeber
- 31: Aufnahme
- 32: Leuchtdioden-Array
- 40: Trenneinrichtung
- 41: Betätigungshebel
- 42: Exzenter
- 43: Hubabschnitt
- 44: Anlagefläche
- 45: Aufnahmestift

## Patentansprüche

1. Anzucht- und Probennahme-Verfahren für Pflanzen, die in einer mehrteiligen Probennahme-Vorrichtung herangezogen werden, wobei die Probennahme-Vorrichtung ein Oberteil (4) mit einer Oberteilkennzeichnung und mit einer Anzahl von Anzuchtbehältern (4.1) sowie ein Unterteil (2) mit einer Unterteilkennzeichnung und mit einer gleichen Anzahl von Probenbehältern (2.1) vorsieht und wobei das Oberteil (4) in einer Zusammenbauposition der Probennahme-Vorrichtung derart mit dem Unterteil (2) verbunden ist, dass die Probenbehälter (2.1) korrespondierend zu den Anzuchtbehältern (4.1) unter ebendiesen angeordnet sind, umfassend die folgenden Schritte:
- während einer Anzuchtphase werden in dem Oberteil (4) der Probennahme-Vorrichtung die Pflanzen in den mit einem Substrat und/oder Nährstoffen gefüllten Anzuchtbehältern (4.1) herangezogen;
- nachdem die Wurzeln der Pflanzen durch eine an jedem Anzuchtbehälter (4.1) vorgesehene Bodenöffnung (4.1.1) in den unterhalb vorgesehenen Probenbehälter (2.1) gewachsen sind, werden in der Zusammenbauposition der Probennahme-Vorrichtung die in dem Unterteil (2) vorgesehenen Wurzelteile mittels eines als Teil der Probennahme-Vorrichtung vorgesehenen und in der Zusammenbauposition an dem Unterteil (2) und/oder dem Oberteil (4) gehaltenen Messers (6) von den Pflanzen abgetrennt, indem das Messer (6) entlang einer an dem Unterteil (2) festgelegten Schneidplatte (8) der Probennahme-Vorrichtung geführt ist;
- in einer einer Analysephase vorausgehenden Aufbereitungsphase wird das Unterteil (2) mit den darin vorgesehenen Wurzelteilen zusammen mit der Schneidplatte (8) von dem Oberteil (4) der Probennahme-Vorrichtung getrennt und dann einer Stanzeinrichtung (20) zugeführt, und dann werden mittels eines Stanzstempels (22) der Stanzeinrichtung (20) rund um Schneidlöcher (8.1) der Schneidplatte (8) ringförmige, bevorzugt kreisringförmige Abschnitte (8.5) aus der Schneidplatte (8) herausgestanzt und in das Innere des jeweils zugeordneten Probenbehälters (2.1) überführt;
- während der Analysephase wird das Unterteil (2) der Probennahme-Vorrichtung mit den darin befindlichen Wurzelteilen einer Analyseeinrichtung zugeführt und für die verschiedenen Wurzelteile wird eine phänotypische Beschreibung und/oder eine molekularbiologische Untersuchung durchgeführt;
- während einer Selektionsphase wird das Oberteil (4) der Probennahme-Vorrichtung mit dem darin befindlichen vitalen Pflanzen einer Selektionseinrichtung zugeführt und positioniert.

2. Anzucht- und Probennahme-Verfahren nach Anspruch 1, **dadurch gekennzeichnet, dass** in der Zusammenbauposition den Anzuchtbehältern (4.1) mit den Bodenöffnungen (4.1.1) zugewandte Probenbehälteröffnungen der Probenbehälter (2.1) von der Schneidplatte (8) überdeckt werden und dass an der Schneidplatte (8) korrespondierend zu der Lage der Bodenöffnungen (4.1.1) und der Probenbehälteröffnungen die Schneidlöcher (8.1) als Durchgangsöffnungen für die Wurzeln der Pflanzen vorgesehen sind.

3. Anzucht- und Probennahme-Verfahren nach Anspruch 1 oder 2, **dadurch gekennzeichnet, dass** das zwischen dem Oberteil (4) und dem Unterteil (2) der Probennahme-Vorrichtung vorgesehene Messer (6) nach Art einer Lochplatte mit einer Anzahl von Schneidlöchern (6.1), welche während der Anzuchtphase korrespondierend zu den Schneidlöchern (8.1) der Schneidplatte (8) so angeordnet sind, dass die Wurzeln in die Probenbehälter (2.1) wachsen können, und dass das Messer (6) zum Durchtrennen der Wurzeln maschinell um einen vorbestimmten Hub in eine Hubrichtung (10) entlang der Schneidplatte (8) geführt wird, wobei der Hub größer gewählt wird als ein Durchmesser der an der Schneidplatte (8) und dem Messer (6) vorgesehenen Schneidlöchern (6.1, 8.1) und kleiner gewählt wird als ein Abstand von zwei in die Hubrichtung (10) benachbarten Probenbehältern (2.1).

4. Anzucht- und Probennahme-Verfahren nach einem der Ansprüche 1 bis 3, **dadurch gekennzeichnet, dass** vor dem Herausstanzen der Abschnitte (8.5) aus der Schneidplatte (8) ein an einem der Schneidplatte (8) zugewandten freien Ende an dem Stanzstempel (22) vorgesehener Positionierkopf (22.2.1) in Eingriff gebracht wird mit den Schneidlöchern (8.1) der Schneidplatte (8) und so eine exakte Lagezuordnung der Schneidplatte (8) und des Unterteils (2) der Probennahme-Vorrichtung zu der Stanzeinrichtung (20) bereitgestellt wird.

5. Anzucht- und Probennahme-Verfahren nach einem der Ansprüche 1 bis 4, **dadurch gekennzeichnet, dass** die ringförmigen Abschnitte (8.5) für verschiedene Probenbehälter (2.1) zeitversetzt in zwei oder mehr Stufen aus der Schneidplatte (8) herausgestanzt werden.

6. Anzucht- und Probennahme-Verfahren nach einem der Ansprüche 1 bis 5, **dadurch gekennzeichnet, dass** die Schneidplatte (8) nach dem Herausstanzen der Abschnitte (8.5) und/oder vor dem Zuführen des Unterteils (2) zu der Analyseeinrichtung von dem Unterteil (2) entfernt wird.

7. Anzucht- und Probennahme-Verfahren nach einem der Ansprüche 1 bis 6, **dadurch gekennzeichnet, dass** in der Selektionsphase ein Anzuchtbehälter (4.1) mittels eines Signalgebers (30) der Selektionseinrichtung gekennzeichnet wird und dass dann jedenfalls ein Teil des darin befindlichen Pflanzensprosses entnommen wird.

8. Anzucht- und Probennahme-Verfahren nach einem der Ansprüche 1 bis 7, **dadurch gekennzeichnet, dass** in der Selektionsphase eine Mehrzahl von Anzuchtbehältern (4.1) eines gleichen Oberteils (4) der Probennahme-Vorrichtung sequenziell mittels des Signalgebers (30) der Selektionseinrichtung gekennzeichnet werden.

9. Anzucht- und Probennahme-Verfahren nach einem der Ansprüche 1 bis 8, **dadurch gekennzeichnet, dass** die Probennahme-Vorrichtung mit den darin vorgesehenen Pflanzen während der Anzuchtphase mittels Leuchtdioden beleuchtet werden.

10. Anzucht- und Probennahme-Verfahren nach einem der Ansprüche 1 bis 9, **dadurch gekennzeichnet, dass** die Anzuchtbehälter (4.1) mit Ziegelgrus als Substrat und/oder Nährstoffen gefüllt und dann während der Anzuchtphase von oben bewässert werden.

11. Anzucht- und Probennahme-Verfahren nach einem der Ansprüche 1 bis 10, **dadurch gekennzeichnet, dass** die Aussaat signalunterstützt erfolgt derart, dass mittels einer Aussaateinrichtung eine auf einer Umverpackung eines Saatguts vorgesehene Saatgutkennzeichnung eingelesen und mittels eines Signalgebers (30) der Aussaateinrichtung ein ausgewählter Anzuchtbehälter (4.1) der Probennahme-Vorrichtung gekennzeichnet wird zur Durchführung der Aussaat.

12. Anzucht- und Probennahme-Verfahren nach einem der Ansprüche 1 bis 11, **dadurch gekennzeichnet, dass** die Analyseeinrichtung und/oder eine zum Betätigen des Messers (6) ausgebildete Trenneinrichtung (0) und/oder die Selektionseinrichtung und/oder die Aussaateinrichtung und/oder die Stanzeinrichtung (20) und/oder der Signalgeber (30) steuerungstechnisch und/oder datentechnisch gekoppelt sind derart, dass die Oberteilkennzeichnung und/oder die Unterteilkennzeichnung und/oder die Saatgutkennzeichnung erfasst werden und mit einem Analyseergebnis der molekularbiologischen Untersuchung der Wurzelteile und insbesondere einer eindeutigen Zuordnung zu einem der Probenbehälter (2.1) und der zugeordneten Anzuchtbehälter (4.1) verknüpft werden.

13. Anzucht- und Probennahme-Verfahren nach einem der Ansprüche 1 bis 12, **dadurch gekennzeichnet, dass** die Aussaateinrichtung und die Selektionseinrichtung einen gemeinsamen Signalgeber (30) verwenden und/oder der Signalgeber (30) der Selektionseinrichtung und/oder der Aussaateinrichtung den Anzuchtbehälter (4.1) und/oder den Probenbehälter (2.1) optisch kennzeichnen.

14. Anzucht- und Probennahme-Verfahren nach einem der Ansprüche 1 bis 13, **dadurch gekennzeichnet, dass** die Probenbehälter (2.1) als Unterteil (2) im 96er Deepwellplatten-Format ausgebildet sind.

## Claims

1. Cultivation and sampling method for plants which are grown in a multi-part sampling device, wherein the sampling device provides an upper part (4) having an upper part designation and having a number of cultivation containers (4.1), as well as a lower part (2) having a lower part designation and an equal number of sample containers (2.1), and wherein when the sampling device is in the assembled position the upper part (4) is connected to the lower part (2) such that the sample containers (2.1) are arranged corresponding to the cultivation containers (4.1) beneath the very same, said method comprising the following steps:
- during a cultivation phase, the plants in the cultivation containers (4.1) filled with a substrate and/or nutrients are grown in the upper part (4) of the sampling device;
- after the roots of the plants have grown through a base opening (4.1.1) provided in each cultivation container (4.1) into the sample container (2.1) provided below, when the sampling device is in the assembled position the root parts provided in the lower part (2) are cut off from the plants by means of a blade (6) provided as part of the sampling device and held in the assembled position on the lower part (2) and/or the upper part (4), in that the blade (6) is guided along a cutting plate (8) of the sampling device fixed on the lower part (2);
- in a preparation phase preceding an analysis phase, the lower part (2) with the root parts provided therein is separated from the upper part (4) of the sampling device together with the cutting plate (8) and is then supplied to a punching device (20), and then annular, preferably circular, sections (8.5) are punched out of the cutting plate (8) by means of a punching stamp (22) of the punching device (20) around cutting holes (8.1) of the cutting plate (8), and said sections are transferred to the interior of the respectively allocated sample container (2.1);
- during the analysis phase, the lower part (2) of the sampling device having the root parts located therein is supplied to an analysis device and for the different root parts a phenotypic description and/or molecular biological examination is carried out;
- during a selection phase, the upper part (4) of the sampling device having the vital plants located therein is supplied to and positioned in a selecting device.

2. Cultivation and sampling method as claimed in claim 1, **characterised in that**, in the assembled position, sample container openings of the sample containers (2.1) facing the cultivation containers (4.1) having the base openings (4.1.1) are covered by the cutting plate (8), and **in that** the cutting holes (8.1) are provided as through-openings for the roots of the plants on the cutting plate (8) corresponding to the position of the base openings (4.1.1) and the sample container openings.

3. Cultivation and sampling method as claimed in claim 1 or 2, **characterised in that** the blade (6), provided between the upper part (4) and the lower part (2) of the sampling device, in the manner of a perforated plate with a number of cutting holes (6.1) which are arranged corresponding to the cutting holes (8.1) of the cutting plate (8) during the cultivation phase such that the roots can grow into the sample containers (2.1), and **in that** the blade (6) is machine-guided by a predetermined stroke in a stroke direction (10) along the cutting plate (8) in order to sever the roots, wherein the stroke is selected to be greater than a diameter of the cutting holes (6.1, 8.1) provided in the cutting plate (8) and on the blade (6) and is selected to be smaller than a distance between two sample containers (2.1) adjacent each other in the stroke direction (10).

4. Cultivation and sampling method as claimed in any one of claims 1 to 3, **characterised in that**, prior to punching out the sections (8.5) from the cutting plate (8), a positioning head (22.2.1) provided on the punching stamp (22) at a free end facing the cutting plate (8) is brought into engagement with the cutting holes (8.1) of the cutting plate (8) and thus a precise positional allocation of the cutting plate (8) and of the lower part (2) of the sampling device to the punching device (20) is provided.

5. Cultivation and sampling method as claimed in any one of claims 1 to 4, **characterised in that** the annular sections (8.5) are punched out of the cutting plate (8) in a time-offset manner in two or more stages for different sample containers (2.1).

6. Cultivation and sampling method as claimed in any one of claims 1 to 5, **characterised in that** the cutting plate (8) is removed from the lower part (2) after punching out the sections (8.5) and/or prior to supplying the lower part (2) to the analysis device.

7. Cultivation and sampling method as claimed in any one of claims 1 to 6, **characterised in that** in the selection phase a cultivation container (4.1) is designated by means of a signal transmitter (30) of the selecting device, and **in that** then at least a part of the plant shoot located therein is removed.

8. Cultivation and sampling method as claimed in any one of claims 1 to 7, **characterised in that** in the selection phase a plurality of cultivation containers (4.1) of the same upper part (4) of the sampling device are designated sequentially by means of the signal transmitter (30) of the selecting device.

9. Cultivation and sampling method as claimed in any one of claims 1 to 8, **characterised in that** the sampling device having the plants provided therein is illuminated during the cultivation phase by means of light-emitting diodes.

10. Cultivation and sampling method as claimed in any one of claims 1 to 9, **characterised in that** the cultivation containers (4.1) are filled with crushed bricks as a substate and/or nutrients and are then watered from above during the cultivation phase.

11. Cultivation and sampling method as claimed in any one of claims 1 to 10, **characterised in that** the sowing is performed in a signal-assisted manner such that, by means of a sowing device, a seed designation provided on a seeds package is read and a selected cultivation container (4.1) of the sampling device is designated by means of a signal transmitter (30) of the sowing device in order to performing sowing.

12. Cultivation and sampling method as claimed in any one of claims 1 to 11, **characterised in that** the analysis device and/or a separating device (0) formed for actuating the blade (6) and/or the selecting device and/or the sowing device and/or the punching device (20) and/or the signal transmitter (30) are coupled via control technology and/or data technology such that the upper part designation and/or the lower part designation and/or the seed designation are detected and are linked with an analysis result of the molecular biological examination of the root parts and in particular a unique allocation to one of the sample containers (2.1) and the allocated cultivation containers (4.1).

13. Cultivation and sampling method as claimed in any one of claims 1 to 12, **characterised in that** the sowing device and the selecting device use a common signal transmitter (30) and/or the signal transmitter (30) of the selecting device and/or the sowing device optically designate the cultivation container (4.1) and/or the sample container (2.1).

14. Cultivation and sampling method as claimed in any one of claims 1 to 13, **characterised in that** the sample containers (2.1) are formed as the lower part (2) in the 96-well Deepwell format.

## Revendications

1. Procédé de culture et de prélèvement d'échantillons pour plantes cultivées dans un dispositif de prélèvement d'échantillons en plusieurs parties, dans lequel le dispositif de prélèvement d'échantillons présente une partie supérieure (4) avec une identification de partie supérieure et avec un certain nombre de récipients de culture (4.1) ainsi qu'une partie inférieure (2) avec une identification de partie inférieure et avec un nombre égal de récipients d'échantillon (2.1), et dans lequel, dans une position de montage du dispositif de prélèvement d'échantillons, la partie supérieure (4) est reliée à la partie inférieure (2) de telle sorte que les récipients d'échantillon (2.1) sont disposés en dessous des récipients de culture (4.1) et en correspondance avec ceux-ci, comprenant les étapes suivantes :
- pendant une phase de culture, les plantes sont cultivées dans les récipients de culture (4.1) remplis d'un substrat et/ou de nutriments dans la partie supérieure (4) du dispositif de prélèvement d'échantillons ;
- après que les racines des plantes ont poussé à travers une ouverture de fond (4.1.1) prévue sur chaque récipient de culture (4.1) dans le récipient d'échantillon (2.1) prévu en dessous, dans la position de montage du dispositif de prélèvement d'échantillons, les parties de racine prévues dans la partie inférieure (2) sont séparées des plantes au moyen d'un couteau (6) prévu comme faisant partie du dispositif de prélèvement d'échantillons et maintenu dans la position de montage sur la partie inférieure (2) et/ou la partie supérieure (4), le couteau (6) étant guidé le long d'une plaque de coupe (8) fixée à la partie inférieure (2) du dispositif de prélèvement d'échantillons ;
- dans une phase de préparation précédant une phase d'analyse, la partie inférieure (2) avec les parties de racine qui y sont prévues est séparée, avec la plaque de coupe (8), de la partie supérieure (4) du dispositif de prélèvement d'échantillons, puis amenée à un moyen de poinçonnage (20) et ensuite, au moyen d'un poinçon (22) du moyen de poinçonnage (20), des sections annulaires, de préférence en forme de couronne, sont poinçonnées dans la plaque de coupe (8) autour de trous de coupe (8.1) de la plaque de coupe (8) et transférées à l'intérieur du récipient d'échantillon (2.1) respectivement associé ;
- pendant la phase d'analyse, la partie inférieure (2) du dispositif de prélèvement d'échantillons avec les parties de racine qui s'y trouvent est amenée à un moyen d'analyse et une description phénotypique et/ou un examen biologique moléculaire sont réalisés pour les différentes parties de racine ;
- pendant une phase de sélection, la partie supérieure (4) du dispositif de prélèvement d'échantillons avec les plantes vitales qui s'y trouvent est amenée à un moyen de sélection et positionnée.

2. Procédé de culture et de prélèvement d'échantillons selon la revendication 1, **caractérisé en ce que**, dans la position de montage, des ouvertures de récipient d'échantillon (2.1) tournées vers les récipients de culture (4.1) munis des ouvertures de fond (4.1.1) sont recouvertes par la plaque de coupe (8) et que les trous de coupe (8.1) sont prévus sur la plaque de coupe (8) comme ouvertures de passage pour les racines des plantes de façon à correspondre à la position des ouvertures de fond (4.1.1) et des ouvertures de récipient d'échantillon.

3. Procédé de culture et de prélèvement d'échantillons selon la revendication 1 ou 2, **caractérisé en ce que** le couteau (6) prévu entre la partie supérieure (4) et la partie inférieure (2) du dispositif de prélèvement d'échantillons est réalisé sous la forme d'une plaque perforée comportant un certain nombre de trous de coupe (6.1) qui, pendant la phase de culture, sont disposés en correspondance avec les trous de coupe (8.1) de la plaque de coupe (8) de telle sorte que les racines puissent pousser dans les récipients d'échantillon (2.1), et que le couteau (6) pour couper les racines est guidé mécaniquement sur une course prédéterminée dans une direction de course (10) le long de la plaque de coupe (8), la course étant choisie pour être supérieure à un diamètre des trous de coupe (6.1, 8.1) prévus sur la plaque de coupe (8) et le couteau (6) et choisie pour être inférieure à une distance entre deux récipients d'échantillon (2.1) adjacents dans la direction de course (10).

4. Procédé de culture et de prélèvement d'échantillons selon l'une des revendications 1 à 3, **caractérisé en ce que**, avant le poinçonnage des sections (8.5) dans la plaque de coupe (8), une tête de positionnement (22.2.1) prévue sur le poinçon (22) et tournée vers la plaque de coupe (8) est mise en prise avec les trous de coupe (8.1) de la plaque de coupe (8) et une association de position exacte de la plaque de coupe (8) et de la partie inférieure (2) du dispositif de prélèvement d'échantillons avec le moyen de poinçonnage (20) est ainsi réalisée.

5. Procédé de culture et de prélèvement d'échantillons selon l'une des revendications 1 à 4, **caractérisé en ce que** les sections annulaires (8.5) pour différents récipients d'échantillon (2.1) sont poinçonnées dans la plaque de coupe (8) en deux ou plusieurs étapes de manière décalée dans le temps.

6. Procédé de culture et de prélèvement d'échantillons selon l'une des revendications 1 à 5, **caractérisé en ce que** la plaque de coupe (8) est retirée de la partie inférieure (2) après le poinçonnage des sections (8.5) et/ou avant l'amenée de la partie inférieure (2) au moyen d'analyse.

7. Procédé de culture et de prélèvement d'échantillons selon l'une des revendications 1 à 6, **caractérisé en ce que**, dans la phase de sélection, un récipient de culture (4.1) est identifié au moyen d'un émetteur de signaux (30) du moyen de sélection et qu'ensuite au moins une partie de la pousse de plante qui s'y trouve est prélevée.

8. Procédé de culture et de prélèvement d'échantillons selon l'une des revendications 1 à 7, **caractérisé en ce que**, dans la phase de sélection, une pluralité de récipients de culture (4.1) d'une même partie supérieure (4) du dispositif de prélèvement d'échantillons sont identifiés séquentiellement au moyen de l'émetteur de signaux (30) du moyen de sélection.

9. Procédé de culture et de prélèvement d'échantillons selon l'une des revendications 1 à 8, **caractérisé en ce que** le dispositif de prélèvement d'échantillons et les plantes qui y sont prévues sont éclairés au moyen de diodes électroluminescentes pendant la phase de culture.

10. Procédé de culture et de prélèvement d'échantillons selon l'une des revendications 1 à 9, **caractérisé en ce que** les récipients de culture (4.1) sont remplis de granules de briques comme substrat et/ou nutriments puis irriguées par le haut pendant la phase de culture.

11. Procédé de culture et de prélèvement d'échantillons selon l'une des revendications 1 à 10, **caractérisé en ce que** l'ensemencement est assisté par signaux de telle sorte qu'une identification de semence prévue sur un emballage d'une semence est lue au moyen d'un moyen d'ensemencement et qu'un récipient de culture sélectionné (4.1) du dispositif de prélèvement d'échantillons est identifié au moyen d'un émetteur de signaux (30) du moyen d'ensemencement pour réaliser l'ensemencement.

12. Procédé de culture et de prélèvement d'échantillons selon l'une des revendications 1 à 11, **caractérisé en ce que** le moyen d'analyse et/ou un moyen de séparation (0) destiné à actionner le couteau (6) et/ou le moyen de sélection et/ou le moyen d'ensemencement et/ou le moyen de poinçonnage (20) et/ou l'émetteur de signaux (30) sont couplés en termes de commande et/ou de données de telle sorte que l'identification de partie supérieure et/ou l'identification de partie inférieure et/ou l'identification de semence sont détectées et liées à un résultat d'analyse de l'examen biologique moléculaire des parties de racine et en particulier à une association univoque à l'un des récipients d'échantillon (2.1) et des récipients de culture associés (4.1).

13. Procédé de culture et de prélèvement d'échantillons selon l'une des revendications 1 à 12, **caractérisé en ce que** le moyen d'ensemencement et le moyen de sélection utilisent un émetteur de signaux commun (30) et/ou l'émetteur de signaux (30) du moyen de sélection et/ou du moyen d'ensemencement identifie optiquement le récipient de culture (4.1) et/ou le récipient d'échantillon (2.1).

14. Procédé de culture et de prélèvement d'échantillons selon l'une des revendications 1 à 13, **caractérisé en ce que** les récipients d'échantillon (2.1) sont réalisés sous la forme d'une partie inférieure (2) au format plaque à 96 puits profonds.
